(19) Europäisches Patentamt / European Patent Office / Office européen des brevets

(11) **EP 0 989 856 B1**

(12) **EUROPÄISCHE PATENTSCHRIFT**

(45) Veröffentlichungstag und Bekanntmachung des Hinweises auf die Patenterteilung:
**12.05.2010 Patentblatt 2010/19**

(21) Anmeldenummer: **98900241.5**

(22) Anmeldetag: **20.01.1998**

(51) Int Cl.:
***A61K 35/12*** (2006.01) ***A61K 31/475*** (2006.01)

(86) Internationale Anmeldenummer:
**PCT/AT1998/000008**

(87) Internationale Veröffentlichungsnummer:
**WO 1998/031378 (23.07.1998 Gazette 1998/29)**

(54) **VERFAHREN UND STOFFE ZUR FREISETZUNG EINES WACHSTUMSFAKTORS AUS ENDOTHELZELLEN, UND NACH DEM VERFAHREN FREIGESETZTER WACHSTUMSFAKTOR SOWIE SEINE VERWENDUNG**

METHOD AND SUBSTANCES FOR RELEASING A GROWTH FACTOR FROM ENDOTHELIAL CELLS, GROWTH FACTOR RELEASED IN ACCORDANCE WITH SAID METHOD AND USE OF SAME

PROCEDE ET SUBSTANCES POUR LIBERER UN FACTEUR DE CROISSANCE ISSU DES CELLULES ENDOTHELIALES, FACTEUR DE CROISSANCE LIBERE SELON CE PROCEDE, ET SON UTILISATION

(84) Benannte Vertragsstaaten:
**AT CH DE FR GB IT LI**

(30) Priorität: **20.01.1997 AT 7397**

(43) Veröffentlichungstag der Anmeldung:
**05.04.2000 Patentblatt 2000/14**

(60) Teilanmeldung:
**09007200.0**
**09007344.6**

(73) Patentinhaber: **IMMODAL PHARMAKA GESELLSCHAFT m.b.H.**
**6111 Volders (AT)**

(72) Erfinder:
- **KEPLINGER, Klaus**
  **A-6020 Innsbruck (AT)**
- **WURM, Martin**
  **A-6020 Innsbruck (AT)**
- **LAUS, Gerhard**
  **A-6020 Innsbruck (AT)**

(74) Vertreter: **Torggler, Paul Norbert et al**
**Patentanwälte Torggler & Hofinger**
**Wilhelm-Greil-Strasse 16**
**Postfach 556**
**6021 Innsbruck (AT)**

(56) Entgegenhaltungen:
**WO-A-82/01130 WO-A-86/00524**
**WO-A-95/21169**

- **REINHARD K.H.: "Uncaria tomentosa (WILLD.) DC.-Cat's Claw, Una de gato oder Katzenkralle" ZEITSCHRIFT FÜR PHYTOTHERAPIE, Bd. 18, Nr. 2, 15.April 1997, Seiten 112-121, XP002064093**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 0 989 856 B1

**Beschreibung**

Seit langem ist bekannt, daß pentazyklische Oxindolalkaloide pharmakologische Wirkungen auf das Immunsystem haben. Eine Steigerung der Phagozytoseleistung der Granulozyten [Wagner H., Kreutzkamp B., Jurcic K., (1985) Planta Med. 51, 419-423] und eine moderate Hemmung der Proliferation leukämischer Zell-Linien [Stuppner H., Sturm S., Geisen G., Zillian U., Konwalinka G. (1993) Planta Med. 59, Suppl. A 583] konnte nachgewiesen werden. An Probanden, die einen alkaloidhaltigen Extrakt aus der Wurzel von *Uncaria tomentosa* (Willd.) DC. eingenommen hatten, wurde eine mit signifikanter Häufigkeit auftretende leichte Lymphozytose beobachtet [Keplinger U. (1995) in *Krallendom: Extrakt aus Radix Uncariae tomentosae (Willd.) DC., Information für Ärzte und Apotheker,* Hrsg: Immodal Pharmaka GmbH, 3. Auflage]. Pentazyklische Oxindolalkaloide weisen demnach eine immunstimulierende, beziehungsweise immunmodulierende Wirkung auf und sind Gegenstand der US-A 5,302,611 [Keplinger K. (1994) *Oxindole alkaloids having properties stimulating the immunologic system and preparations containing the same*] oder der WO-A 86/00524 [Keplinger K., Wagner H., Kreutzkamp B. (1986) *Oxindolalkaloide mit immunsystem-stimulierenden Eigenschaften und diese enthaltendes Präparat*].

Es ist bekannt, daß tetrazyklische Oxindolalkaloide auf das Zentralnervensystem wirken, negativ chronotrope und negativ inotrope Effekte auslösen [Kanatani H., Kohda H., Yamasaki K., Hotta I., Nakata Y., Segawa T., Yamanaka E., Aimi N., Sakai S.I. (1984) J. Pharm. Pharmacol. 37, 401-404; Zhang W., Liu G.X. (1986) Act. Pharmacol. Sinica 7 (5), 426-428; Zhu Y., Guoxiong H.X. (1993) Chin. J. Pharmacol. Toxicol. 7 (2), 117-121], den $Ca^{2+}$-Transport blockieren [Sun A., Liu G., Wang X., Zhang W., Huang X. (1988) Chin. J. Pharmacol. Toxicol. 2 (2), 93-97; Zhang W., Liu G., Huang X. (1987) Act. Pharmacol. Sinica 8, 425-429] und die Aggregation der Blutplättchen behindern [Jin R.M., Chen C.X., Li Y.K., Xu P.K. (1991) Act. Pharmaceut. Sinica 26 (4), 246-249; Chen C.X., Jin R.M., Li Y.K., Zhong J., Yue L., Chen S.C., Zhou J.Y. (1992) Act. Pharmacol. Sinica 13 (2), 126-130].

Seit langem ist auch bekannt, daß Oxindolalkaloide in Lösung isomerisieren. Über die Gesetzmäßigkeiten der Isomerisierung wurde erst in letzter Zeit berichtet [Laus G., Brössner D., Senn G., Wurst K. (1996) J.Chem.Soc., Perkin Trans. 2, 1931-1936]. Die Herstellung definierter Isomerengemische ist aus der AT-B 400 950 bekannt [Keplinger K., Keplinger D., Laus G. (1996) *Verfahren zur technischen Herstellung definierter Isomerengemische aus Verbindungen mit spirozyklischen Aminocarboxyl- und/oder spirozyklischen Aminocarbonyl-Systemen*].

Die für die vorliegenden Arbeiten verwendeten Alkaloide wurden aus der Wurzel von *Uncaria tomentosa* gewonnen. Gleichzeitig wurde an einer Reihe von Pflanzen dieser Art das Alkaloidmuster studiert. Dabei wurde festgestellt, daß *Uncaria tomentosa* in zwei Modifikanten in der Natur vorkommt und daß sich gelegentlich eine Modifikante in die andere umwandelt. Unter Modifikation versteht man die Ausbildung unterschiedlicher Merkmale (Blütenfarbe, Blattform, Inhaltsstoffe, Geschlecht) aufgrund unterschiedlicher Umwelteinflüsse (Licht, Temperatur, Nährstoffe, Pilzbefall) ohne Veränderung des Erbgutes. Bei *Uncaria tomentosa* enthält eine Modifikante, die mit *mod. tetr.* bezeichnet wird, überwiegend die tetrazyklischen Oxindolalkaloide Rhynchophyllin und Isorhynchophyllin, die andere, die *mod. pent.* genannt wird, die pentazyklischen Oxindolalkaloide Pteropodin, Isopteropodin, Speciophyllin und Uncarin F, sowie Mitraphyllin und Isomitraphyllin [Laus G., Brössner D., Keplinger K. (1997) Phytochemistry 45, 855-860]. Es gibt auch Übergangsformen, die beide Alkaloidtypen in unterschiedlichen Mischungsverhältnissen enthalten [Laus G., Keplinger D. (1994) J. Chromatogr. A 662, 243-249]. Bei den im folgenden beschriebenen Untersuchungen wurden sowohl tetrazyklische als auch pentazyklische Oxindolalkaloide verwendet.

Allgemeine Struktur der pentazyklischen Oxindolalkaloide mit Notation der Stereochemie :

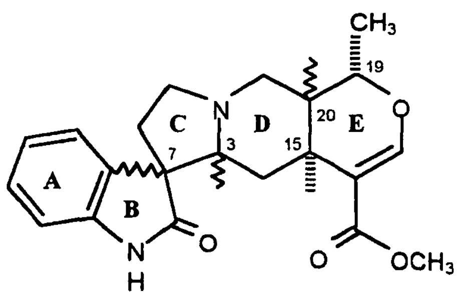

| | | |
|---|---|---|
| 1 | Pteropodin | 3*S*, 7*R*, 15*S*, 19*S*, 20*S* |
| 2 | Isopteropodin | 3*S*, 7*S*, 15*S*, 19*S*, 20*S* |
| 3 | Speciophyllin | 3*R*, 7*S*, 15*S*, 19*S*, 20*S* |

(fortgesetzt)

| | | |
|---|---|---|
| 4 | Uncarin F | 3*R*, 7*R*, 15*S*, 19*S*, 20*S* |
| 5 | Mitraphyllin | 3*S*, 7*R*, 15*S*, 19*S*, 20*R* |
| 6 | Isomitraphyllin | 3*S*, 7*S*, 15*S*, 19*S*, 20*R* |

Allgemeine Struktur der tetrazyklischen Oxindolalkaloide mit Notation der Stereochemie :

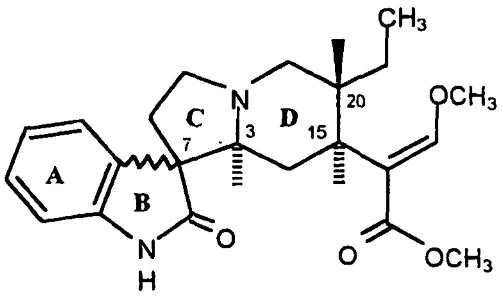

| | | |
|---|---|---|
| 7 | Rhynchophyllin | 3*S*, 7*R*, 115*S*, 20*R* |
| 8 | Isorhynchophyllin | 3*S*, 7*S*, 15*S*, 20*R* |

Um beim Sammeln von Pflanzenmaterial vor Ort zumindest eine qualitative Unterscheidung zwischen den beiden Modifikanten von Uncaria tomentosa zu ermöglichen, wurde eine dünnschichtchromatographische Methode entwickelt [Laus G., Keplinger K. (1997) Zeitschrift f. Phytotherapie, 122-126], die anhand der Fig. 1 näher beschrieben wird: Zur Identitätsprüfung des Drogenmaterials eignet sich die Dünnschichtchromatographie ausgezeichnet, insbesondere wenn man die charakteristische Eigenschaft der Oxindol-Alkaloide, in wäßrigen Lösungen pH-abhängig zu isomerisieren, als zusätzliches Kriterium anwendet. Als Referenzsubstanz schlagen wir Ajmalicin vor, weil es eine verwandte Struktur besitzt und käuflich ist. Sein $R_f$-Wert ist etwas höher als der von Isopteropodin. Um Schwankungen in den chromatographischen Bedingungen auszugleichen, werden alle Werte auf die Referenzsubstanz bezogen ($hR_{Ajmalicin}$-Werte).

1 g Droge wird mit 50ml destilliertem Wasser 45 Minuten auf 85°C erhitzt. Dieser Extrakt wird dekantiert, die Droge noch einmal mit 20 ml Wasser nachgewaschen und das Waschwasser mit dem Extrakt vereinigt. Diese vereinigten Extrakte werden in zwei gleich große Portionen geteilt. Ein Teil wird mit 1 Tropfen Salzsäure 7 % versetzt (ca. pH 4, Lösungen 1, 3 bzw. 6) und 24 h unter Rückfluß gekocht, der andere Teil wird mit 1 Tropfen Natriumhydroxidlösung 8.5 % versetzt (ca. pH 8, Lösungen 2, 4 bzw. 7) und 24 h auf 50°C erwärmt. Nach dem Abkühlen auf Raumtemperatur gibt man zur sauren Lösung 2 Tropfen Natriumhydroxidlösung 8.5 %. Die beiden Lösungen werden jeweils mit 3 x 5 ml Chloroform extrahiert, wobei bei jedem Extraktionsschritt mindestens 4 ml organische Phase gesammelt werden müssen. Die Extrakte werden durch Zugabe von je einer Spatelspitze wasserfreies Natriumsulfat getrocknet und zur Trockne eingedampft. Die Rückstände werden in 0.5 ml Chloroform gelöst und diese Lösungen für die Chromatographie verwendet. Als Referenzlösung wird 1 mg Ajmalicin (Fluka) in 1 ml Chloroform gelöst (Lösung 5).

Je 10 µl werden auf DC-Plastikfolien (Kieselgel 60 $F_{254}$, 20 x 20 cm, 0.2 mm Schichtdicke; Merck Art. Nr. 1.05735.0001) punktförmig aufgetragen. Als Fließmittel wird Ethylacetat / n-Hexan (95:5) verwendet. Zur Detektion wird die Fluoreszenzminderung bei 254 nm herangezogen.

| Alkaloid | $hR_{Ajmalicin}$-Werte |
|---|---|
| Speciophyllin | 12 |
| Mitraphyllin | 26 |
| Uncarin F | 56 |
| Isomitraphyllin | 75 |
| Pteropodin | 83 |
| Isopteropodin | 95 |

(fortgesetzt)

| Alkaloid | *h*$R_{Ajmalicin}$-Werte |
|---|---|
| Rhynchophyllin | 22 |
| Isorhynchophyllin | 91 |
| Ajmalicin | 100 |

Bei *U.tomentosa mod. pent.* (Reihe 1 und 2) sind die Spots von 6 Alkaloiden zu erkennen. In der sauer isomerisierten Lösung 1 erscheinen Speciophyllin, Mitraphyllin und Pteropodin stärker, während in der alkalisch isomerisierten Lösung 2 Isopteropodin und Isomitraphyllin stärker hervortreten. Die Lösungen 3 und 4 enthalten Rhynchophyllin und Isorhynchophyllin aus *U.tomentosa mod. tetr.* und zeigen eine ähnliche Abhängigkeit vom pH wie Mitraphyllin und Isomitraphyllin. Lösung 5 dient als Referenz. Die Lösungen 6 und 7 stammen von einer typischen Mischdroge, in der die unerwünschten Alkaloide Rhynchophyllin und Isorhynchophyllin deutlich zu erkennen sind. Ein HPLC-Chromatogramm, das die Bestimmung der pentazyklischen neben den tetrazyklischen Alkaloiden erlaubt, wie in Ausführungsbeispiel 8 beschrieben, ist in Fig. 2 abgebildet.

Da Oxindolalkaloide in wässeriger Lösung isomerisieren, wurden für die folgenden Untersuchungen nicht einzelne Isomere, sondern die Gruppen der zusammengehörenden Isomeren eingesetzt. Untersucht wurde zunächst ein Standard-Alkaloidgemisch wie es in der Wurzel von Uncaria tomentosa mod. pent. gefunden wird (IMM-2414), in weiterer Folge die Isomerengruppen des Mitraphyllins (IMM-2417), des Rhynchophyllins (IMM-2418) und des Pteropodins (IMM-2435). Die Zusammensetzung der verwendeten Gemische ist in nachstehender Tabelle 1 beschrieben. Auch einfache Derivate wurden untersucht, nämlich Alkaloid-Carbonsäuren (IMM-2413), die aus dem Standardgemisch (IMM-2414) durch alkalische Hydrolyse hergestellt wurden, und Alkaloid-N-Oxide (IMM-2433), die durch Oxidation mit Wasserstoffperoxid aus dem Standardgemisch (IMM-2414) erzeugt wurden.

Tabelle 1 : Zusammensetzung der untersuchten Alkaloidgemische

| Alkaloid | Bezeichnung IMM-2414 | IMM-2417 | IMM-2418 | IMM-2435 |
|---|---|---|---|---|
| Speciophyllin | 4% | ---- | ---- | 4% |
| Uncarin F | 6% | ---- | ---- | 6% |
| Pteropodin | 28 % | ---- | ---- | 30 % |
| Isopteropodin | 57 % | ---- | ---- | 60 % |
| Mitraphyllin | 2% | 33 % | ---- | ---- |
| Isomitraphyllin | 3% | 67 % | ---- | ---- |
| Rhynchophyllin | ---- | ---- | 40 % | ---- |
| Isorhynchophyllin | ---- | ---- | 60 % | ---- |

Erklärung : Die Zusammensetzung der Alkaloidgemische wurde durch HPLC-Analyse bestimmt. Die Prozentangaben stellen Gewichtsprozente dar.

## Die Verteilung der Alkaloide in biologischen Systemen

Zellen in einer Nährlösung können als ein Zwei-Phasen-System aus Wasser und Lipiden betrachtet werden. Untersuchungen zur Verteilung der Alkaloide in Zellkulturen und in einfachen Octanol-Wasser-Gemischen wurden durchgeführt. Dabei wurde festgestellt, daß die einzelnen Isomeren unterschiedliches Verteilungsverhalten aufweisen, wie Tabelle 2 zeigt.

Tabelle 2 : Logarithmen der Verteilungskoeffizienten $K_{O/W}$ bei pH 7

| Alkaloid | log $K_{O/W}$ |
|---|---|
| Pteropodin | 2.9 |
| Isopteropodin | 3.2 |
| Speciophyllin | 1.4 |
| Uncarin F | 3.1 |
| Mitraphyllin | 2.5 |
| Isomitraphyllin | 2.8 |

(fortgesetzt)

| Alkaloid | log $K_{O/W}$ |
|---|---|
| Rhynchophyllin | 2.7 |
| Isorhynchophyllin | 3.1 |

Die Alkaloide wurden zwischen gleichen Volumina von Octanol und wässerigem Phosphatpuffer pH 7 (0.01 M) bei 20 °C verteilt. Die Konzentrationen c der Alkaloide in den 2 Phasen wurden mittels HPLC bestimmt und daraus die Verteilungskoeffizienten

$$K_{O/W} = \frac{c\ \text{Octanol}}{c\ \text{Wasser}}$$

berechnet. Es war zu erwarten, daß das Isomerengleichgewicht in einem Zwei-Phasen-System (Fig. 3) nicht nur vom pH-Wert der Wasserphase, sondern auch von der Art und der Menge der organischen Phase bestimmt wird. Am Beispiel von Mitraphyllin und Isomitraphyllin wurden die Gleichgewichte in einem Octanol-Wasser(1:1)-System bei pH-Werten von 3 bis 7 untersucht (Fig. 4). Aus der Abbildung ist ersichtlich, daß im pH-Bereich von 4 bis 7 überwiegend Isomitraphyllin in der Octanol-Phase vorliegt, mit einem Maximum von 94 Mol.% bei etwa pH 5. Unterhalb von pH 3 beginnt hingegen Mitraphyllin in der wäßrigen Phase zu dominieren. Bei keinem pH-Wert wird Isomitraphyllin in Wasser in größerem Ausmaß gebildet. Die Situation der Isomerenverteilung in einem 2-Phasen-System unterscheidet sich also deutlich von der in einer rein wäßrigen Lösung.

In EA.hy926- Endothelzellkulturen, die mit verschiedenen Alkaloidgemischen ($c \approx 1\ \mu M$) inkubiert wurden, konnte eine Abnahme der Konzentrationen der pentazyklischen Iso-Alkaloide, Isopteropodin, und Isomitraphyllin nach 7 Tagen beobachtet werden, während die Konzentration von Isorhynchophyllin und den anderen Isomeren praktisch unverändert blieb oder leicht anstieg, wie dies aus Tabelle 3 ersichtlich ist. Als Medium wurde RPMI-1640 (komplettiert mit 10 Vol.% fötalem Kälberserum, 2 mM Glutamin, 50 Einheiten/ml Penicillin G und 50 µg/ml Streptomycin) verwendet.

Tabelle 3 : Abnahme der Alkaloidkonzentrationen (mg / l) im Medium von EA.hy926-Endothelzellkulturen nach 7 Tagen

| Stimulans (Konzentration) | Alkaloid | Start | 7 Tage |
|---|---|---|---|
| IMM-2414 (1.0 µM) | Isopteropodin | 0.22 | 0.12 |
| | Pteropodin | 0.10 | 0.16 |
| | Summe | 0.32 | 0.28 |
| IMM-2417 (1.0 µM) | Isomitraphyllin | 0.28 | 0.14 |
| | Mitraphyllin | 0.10 | 0.11 |
| | Summe | 0.38 | 0.25 |
| IMM-2418 (1.1 µM) | Isorhynchophyllin | 0.27 | 0.27 |
| | Rhynchophyllin | 0.15 | 0.13 |
| | Summe | 0.42 | 0.40 |
| IMM-2435 (1.4 µM) | Isopteropodin | 0.35 | 0.18 |
| | Pteropodin | 0.11 | 0.20 |
| | Summe | 0.46 | 0.38 |

Erklärung :
IMM-2414 = Lösung des Alkaloid-Standardgemisches in Medium
IMM-2417 = Lösung des Isomerengemisches des Mitraphyllins in Medium
IMM-2418 = Lösung des Isomerengemisches des Rhynchophyllins in Medium
IMM-2435 = Lösung des Isomerengemisches des Pteropodins in Medium

Die unterschiedlichen Konzentrationsänderungen sind mit der Löslichkeit der Alkaloide nicht zu erklären. Die Abnahme der Alkaloidkonzentration muß also eine Folge physiologischer Vorgänge im Zytosol sein, in dessen Verlauf die pentazyklischen Oxindolalkaloide metabolisiert, also verbraucht werden. Die Isomerisierung der Oxindolalkaloide verläuft in Gegenwart der Endothelzellen anders als im reinen Medium. In den sieben Tagen eines Versuchsansatzes

entsteht im Wechselspiel zwischen physikalisch bedingter und physiologisch induzierter Isomerisierung ein Isomerengemisch, dessen Bestandteile einzeln oder zusammen wirksam sein können.

Pteropodin und Isopteropodin, beziehungsweise Mitraphyllin und Isomitraphyllin, finden sich dann in dem für den pH-Wert des Mediums (EA.hy926-Endothelzellen in RPMI-1640 : pH 7.5 beim Start, pH 8.1 nach 7 Tagen) untypischen Verhältnis von annähernd 1:1, wohingegen Rhynchophyllin und Isorhynchophyllin auch in Gegenwart von Endothelzellen unbeeinflußt zu dem für den pH-Wert typischen Gleichgewicht von 1:2 isomerisieren. Die Aktivität von einzelnen Isomeren kann mit diesem Modell, in dem immer Isomerisierung eintritt, natürlich nicht bestimmt werden. Es kann aber festgestellt werden, daß bei den pentazyklischen Oxindolalkaloiden ein Umsatz stattfindet, bei den tetrazyklischen jedoch nicht.

## Die Untersuchungen an Zellkulturen

Die Substanzen wurden an Endothelzellen untersucht, weil für solche Zellen Wechselwirkungen mit immunologischen Reaktionen bekannt sind [Kirchner H., Kruse A., Neustock P., Rink L., (1993) Cytokine und Interferone: Botenstoffe des Immunsystems, 61]. Dabei wurde erkannt, daß sowohl transformierte EA.hy926-Endothelzellen [Edgell C.-J.S., McDonald C.C., Graham J.B. (1983) Proc. Natl. Acad. Sci. USA 80, 3734-3737] als auch normale humane Nabelschnur-Endothelzellen (HUVEC, ATCC CRL-1730) unter dem Einfluß der pentazyklischen Alkaloide (c = 1 μM) einen Wachstumsfaktor in das Medium abgeben, der die Proliferation von Lymphozyten in signifikantem Ausmaß beeinflußt. Für EA. hy926-Endothelzellen wurde als Medium RPMI-1640 (komplettiert mit 10 Vol.% fötalem Kälberserum, 2 mM Glutamin, 50 Einheiten/ml Penicillin G und 50 μg/ml Streptomycin) und für die Nabelschnur-Endothelzellen als Medium HAM F12 (komplettiert 10 Vol.% fötalem Kälberserum 60 μg/ml EndothelialCellGrowthStimulant und 100 μg/ml Heparin) verwendet.

Daraufhin wurde die Wirkung dieses Wachstumsfaktors auf Lymphozyten näher untersucht. Es wurde festgestellt, daß dieser Faktor auf immortalisierte (z.B. die EBV-transformierte lymphoblastoide Zell-Linie Raji und die leukämische Zell-Linie Jurkat) und auf normale humane B- und T-Lymphozyten (isoliert aus Vollblut normaler Spender) in verschiedener Weise wirkt.

Für die Untersuchung von nomalen, humanen, schwach oder nicht aktivierten B- und T-Lymphozyten wurden die Überstände (SN = supernatant) von mit IMM-2414 7 Tage stimulierten Endothelzellkulturen (SN-2414) und nicht stimulierten Endothelzellkulturen (SN-Medium) in verschiedenen Konzentrationen zu Lymphozytenkulturen gegeben. Eine Steigerung der Proliferation der Lymphozyten wurde nach 5 Tagen durch Messung des [$^3$H]Thymidin-Einbaus festgestellt, wie sich aus der Tabelle 4 ergibt. Dazu wurden die normalen Zellen 18 Stunden mit 1 μCi [$^3$H]Thymidin behandelt, auf Nitrozellulose geerntet und anschließend die Radioaktivität im Szintillationszähler (cpm = counts per minute) gemessen. Jeder Test wurde dreifach durchgeführt.

Tabelle 4 : Proliferation (cpm nach [$^3$H]Thymidin-Einbau) von normalen humanen, nicht oder schwach aktivierten B- und T-Lymphozyten in Medium RPMI-1640

| Stimulans (Verdünnung) | B-Lymphozyten | | | T-Lymphozyten | | |
|---|---|---|---|---|---|---|
| Medium | 363 | ± 213 | | 349 | ± 114 | |
| IMM-2414 (1 μM) | 352 | ± 323 | | 332 | ± 82 | |
| und unter dem Einfluß von EA.hy926-Endothelzellkultur-Überständen | | | | | | |
| SN-Medium (1:4) | 1015 | ± 618 | | 591 | ± 252 | |
| SN-2414 (1:4) | 1527 | ± 540 | | 1242 | ± 752 | *** |
| SN-Medium (1:8) | 1381 | ± 390 | | 493 | ± 278 | |
| SN-2414 (1:8) | 2039 | ± 530 | *** | 1084 | ± 549 | **** |
| SN-Medium (1:16) | 1263 | ± 299 | | 371 | ± 151 | |

(fortgesetzt)

| | | | | | | |
|---|---|---|---|---|---|---|
| und unter dem Einfluß von EA.hy926-Endothelzellkultur-Überständen | | | | | | |
| SN-2414 (1: 16) | 1795 | ± 584 | * | 549 | ± 250 | ** |

Erklärung :
Medium = RPMI-1640, komplettiert wie vorstehend angegeben.
IMM-2414 = Lösung des Alkaloid-Standardgemisches in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
Die Werte stellen den Mittelwert ± Standardabweichung von mindestens 7 Experimenten dar. Die Signifikanz wurde mittels Student t-Test durch paarweisen Vergleich mit der Kontrollgruppe (SN-Medium) ermittelt:
* $P < 0.05$, ** $P < 0.01$, *** $P < 0.005$, **** $P < 0.001$.

Man erkennt, daß die Alkaloide allein keinen Einfluß im Vergleich zum Medium ausüben. Die Überstände der nicht stimulierten Endothelzellkulturen (SN-Medium) führen schon zu einer Steigerung der Proliferation, die Überstände von mit IMM-2414 stimulierten Endothelzellkulturen (SN-2414) verstärken diese Steigerung noch deutlich. Im Fall der B-Lymphozyten erkennt man den maximalen Effekt bei einer Verdünnung des Überstandes SN-2414 mit dem gleichen Medium von 1:8, im Fall der T-Lymphozyten bei einer Verdünnung von 1:4. (Fig. 5)

Für die Untersuchung von transformierten Zellen (Raji ATCC CCL86 und Jurkat ATCC E6.1) wurden die Überstände von mit IMM-2414 7 Tage stimulierten Endothelzellkulturen (SN-2414) und nicht stimulierten Endothelzellkulturen (SN-Medium) in verschiedenen Konzentrationen zu den Zellkulturen gegeben. Im Gegensatz zu den oben erwähnten B- und T-Lymphozyten wurde hier eine Hemmung der Proliferation der transformierten Zellen festgestellt, wie Tabelle 5 zeigt. Die Messung des [$^3$H]Thymidin-Einbaus erfolgte nach 2 Tagen. Weiters wurden auch Zellkulturen der myeloiden Zell-Linie U937 ATCC CRL1593.2 untersucht. Die transformierten Zellen wurden 5 Stunden mit 0.5 µCi [$^3$H]Thymidin behandelt, auf Nitrozellulose geerntet und anschließend die Radioaktivität im Szintillationszähler (cpm = counts per minute) gemessen.

Tabelle 5 : Proliferation (cpm nach [$^3$H]Thymidin-Einbau) verschiedener Zell-Linien in Medium RPMI-1640

| Stimulans (Verdünnung) | Raji CCL86 [a] | | | Jurkat E6.1 [a] | | | U937 CRL-1593.2 [b] | |
|---|---|---|---|---|---|---|---|---|
| Medium | 21621 | ± 5755 | | 35085 | ± 13876 | | 121349 | ± 18653 |
| IMM-2414 (1 µM) | 21698 | ± 6299 | | 35688 | ± 14020 | | 123346 | ± 26412 |
| und unter dem Einfluß von EA.hy926-Endothelzellkultur-Überständen | | | | | | | | |
| SN-Medium (1:2) | 32967 | ± 9652 | | 26544 | ± 17492 | | 86115 | ± 30792 |
| SN-2414 (1:2) | 4801 | ± 3766 | **** | 4282 | ± 3186 | ** | 84736 | ± 33654 |
| SN-Medium (1:4) | 30919 | ± 11134 | | 34716 | ± 17394 | | 101093 | ± 24813 |
| SN-2414 (1:4) | 9178 | ± 7671 | **** | 7163 | ± 6268 | *** | 94257 | ± 30331 |
| SN-Medium (1:8) | 21976 | ± 7443 | | 41428 | ± 16648 | | 118634 | ± 19980 |

(fortgesetzt)

| | | | | | | | | |
|---|---|---|---|---|---|---|---|---|
| und unter dem Einfluß von EA.hy926-Endothelzellkultur-Überständen | | | | | | | | |
| SN-2414 (1:8) | 11357 | ± 5308 | * | 8044 | ± 3921 | *** | 107771 | ± 30975 |

Erklärung :
Medium = RPMI-1640, komplettiert wie vorstehend angegeben.
IMM-2414 = Lösung des Alkaloid-Standardgemisches in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
Die Werte stellen den Mittelwert ± Standardabweichung von mindestens [a] 7 Experimenten, oder [b] 3 Experimenten dar. Die Signifikanz wurde mittels Student t-Test durch paarweisen Vergleich mit der Kontrollgruppe (SN-Medium) ermittelt :
* $P < 0.05$, ** $P < 0.01$, *** $P < 0.005$, **** $P < 0.001$.

Man erkennt, daß die Alkaloide allein keinen Einfluß im Vergleich zum Medium ausüben. Die Überstände der mit IMM-2414 stimulierten Endothelzellkulturen (SN-2414) hemmen die Proliferation der Raji- und Jurkat-Zellen. Man erkennt die Dosisabhängigkeit des Effekts deutlich, während im Fall der myeloiden U937-Zellen keine Beeinflussung sichtbar ist (Fig. 6).

In einer Untersuchung von hoch aktivierten B- und T-Lymphozyten (aus peripherem Blut oder Tonsillen) wurden die Überstände von mit IMM-2414 7 Tage stimulierten Endotheizellkulturen (SN-2414) und nicht stimulierten Endothelzellkulturen (SN-Medium) in verschiedenen Konzentrationen zu den Kulturen hochaktivierter Lymphozyten (Lymphoblasten) gegeben. Auch hier wurde eine Hemmung der Proliferation der Lymphozyten durch Messung des [$^3$H]Thymidin-Einbaus festgestellt, wie Tabelle 6 zeigt.

Tabelle 6 : Proliferation (cpm nach [$^3$H]Thymidin-Einbau) von hochaktivierten humanen B- und T-Lymphozyten (Lymphoblasten) in Medium RPMI-1640

| Stimulans (Verdünnung) | B-Lymphoblasten | T-Lymphoblasten |
|---|---|---|
| Medium | 17840 | 2186 |
| IMM-2414 (1 μM) | 16610 | 2097 |
| und unter dem Einfluß von EA.hy926-Endothelzellkultur-Überständen | | |
| SN-Medium (1:8) | 9594 | 1254 |
| SN-2414 (1:8) | 1527 | 884 |
| SN-Medium (1:16) | 13865 | 1554 |
| SN-2414 (1:16) | 1699 | 728 |
| SN-Medium (1:32) | 13903 | 2049 |
| SN-2414 (1:32) | 2534 | 720 |

Erklärung :
Medium = RPMI-1640, komplettiert wie vorstehend angegeben.
IMM-2414 = Lösung des Alkaloid-Standardgemisches in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
Die Werte stellen Ergebnisse von Einzelexperimenten dar.

Man erkennt, daß die Alkaloide allein keinen Einfluß im Vergleich zum Medium ausüben. Die Überstände der nicht stimulierten Endothelzellkulturen (SN-Medium) führen schon zu einer Hemmung der Proliferation, die Überstände von mit IMM-2414 stimulierten Endothelzellkulturen (SN-2414) verstärken diese Hemmung noch deutlich (Fig. 7). Die Dosisabhängigkeit des Effekts ist deutlich erkennbar.

Weiters wurden Überstände von mit IMM-2414, IMM-2417 oder IMM-2435 7 Tage stimulierten Endothelzellkulturen (SN-2414, SN-2417, SN-2435) und nicht stimulierten Endothelzellkulturen (SN-Medium) in verschiedenen Konzentrationen zu Kulturen hochaktivierter Lymphozyten (Lymphoblasten) gegeben (Tabelle 7).

Tabelle 7 : Proliferation (cpm nach [$^3$H]Thymidin-Einbau) von humanen hoch aktivierten T-Lymphozyten (Lymphoblasten) in Medium RPMI-1640

| Stimulans (Verdünnung) | T-Lymphoblasten |
|---|---|
| Medium | 4065 |
| IMM-2414 | 4557 |
| IMM-2417 | 3929 |
| IMM-2435 | 4124 |
| und unter dem Einfluß von EA.hy926-Endothelzell-Überständen | |
| SN-Medium (1:4) | 3113 |
| SN-2414 (1:4) | 652 |
| SN-2417 (1:4) | 2285 |
| SN-2435 (1:4) | 1887 |
| SN-Medium (1:8) | 3139 |
| SN-2414 (1:8) | 2628 |
| SN-2417 (1:8) | 1595 |
| SN-2435 (1:8) | 2380 |
| SN-Medium (1:16) | 2320 |
| SN-2414 (1:16) | 1852 |
| SN-2417 (1:16) | 1913 |
| SN-2435 (1:16) | 1821 |

Erklärung:
Medium = RPMI-1640, komplettiert wie vorstehend angegeben.
IMM-2414 = Lösung des Alkaloid-Standardgemisches in Medium
IMM-2417 = Lösung des Isomerengemisches des Mitraphyllins in Medium
IMM-2435 = Lösung des Isomerengemisches des Pteropodins in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
SN-2417 = Überstand der 7 Tage mit dem Isomerengemisch des Mitraphyllins in Medium stimulierten Endothelzellenkultur, verdünnt mit gleichem Medium im angegebenen Verhältnis.
SN-2435 = Überstand der 7 Tage mit dem Isomerengemisch des Pteropodins in Medium stimulierten Endothelzellenkultur, verdünnt mit gleichem Medium im angegebenen Verhältnis.
Die Werte stellen Ergebnisse von Einzelexperimenten dar.

Man erkennt, daß die Alkaloide allein keinen Einfluß im Vergleich zum Medium ausüben. Die Überstände der nicht stimulierten Endothelzellkulturen (SN-Medium) führen schon zu einer Hemmung der Proliferation, die Überstände von mit IMM-2414, IMM-2417 oder IMM-2435 stimulierten Endothelzellkulturen (SN-2414, SN-2417, SN-2435) verstärken diese Hemmung noch deutlich (Fig. 8). Die Dosisabhängigkeit des Effekts ist deutlich erkennbar.

Untersuchungen von HIV-infizierten humanen Lymphozyten zeigten ebenfalls, wie die permanenten Zell-Linien Raji und Jurkat unter dem Einfluß des Wachstumsfaktors einen Rückgang ihrer Proliferation (Tabelle 8) und eine Verminderung der Virus-Replikation, gemessen an der Verminderung der $p^{24}$-Antigen-Synthese, um etwa 50%, wie aus der Tabelle 9 ersichtlich ist. Der experimentelle Ansatz war wie folgt: Humane Lymphozyten wurden für zwei Tage mit Phytohämagglutinin stimuliert. Anschließend wurden die Zellen gewaschen und mit HIV-1, Stamm IIIB in einer m.o.i. von 0,0001 infiziert (60 min/37°C). Nach Auswaschen des überschüssigen Inoculums wurden die infizierten Zellen zu den Testsubstanzen gegeben. Der Gesamtansatz wurde für 4 Tage bei 37°C, 5% $CO_2$ und 95% Luftfeuchtigkeit inkubiert.

Anschließend wurde in den Kulturüberständen die Menge an de-novo-synthetisiertem HIVcore-Protein p24 mit Hilfe eines Sandwich ELISA bestimmt. Die Quantifizierung erfolgte über eine Eichkurve mit rekombinantem p24: die Ergebnisse sind als ng p24/ml Kulturüberstand angegeben. Die proliferative Aktivität der infizierten Zellen, die in Gegenwart der Testsubstanzen kultiviert wurden, wurde mittels "MTT-Test" bestimmt. Es wurde die prozentuale Proliferation gegenüber einer unbehandelten Kontrolle errechnet. Alle Kulturen wurden im Triplikat angesetzt.

Während mit der antiretroviralen Referenzsubstanz Azidothymidin (AZT) eine dosisabhängige Hemmung der HIV-Replikation erzielt wurde, bewirkte der Zellkulturüberstand SN-2414 in der höchsten getesteten Konzentration eine etwa 50%-ige Hemmung der HIV-Replikation. Zudem hat die Probe in dieser Konzentration einen antiproliferativen Effekt.

Tabelle 8

| | | Proliferation (% der Kontrolle) | | |
|---|---|---|---|---|
| Verdünnung | | 1:4 | 1:8 | 1:16 |
| Substanzen: | IMM-2414 (1 μM) | 99 | 100 | 100 |
| | LPS (10 μg/ml) | 100 | 100 | 100 |
| Überstände: | Kontrolle | 100 | 100 | 100 |
| | SN-2414 | 86 | 100 | 100 |
| | LPS | 100 | 100 | 100 |

Erklärung:
IMM-2414 = Lösung des Alkaloid-Standardgemisches in Medium
SN-2414 = Überstand der 7 Tage mit dem Alkaloid-Standardgemisch in Medium stimulierten Endothelzellenkultur, verdünnt mit gleichem Medium im angegebenen Verhältnis.
LPS = Lipopolysaccharid

Tabelle 9

| | | HIV-Replikation in vitro | | |
|---|---|---|---|---|
| Verdünnung | | 1:4 | 1:8 | 1:16 |
| Substanzen: | IMM-2414 (1 μM) | 68,2 | 59,1 | 59,2 |
| | LPS (10 μg/ml) | 65,2 | 57,5 | 56,3 |
| Überstände: | Kontrolle | 53,9 | 62,6 | 62,7 |
| | SN-2414 | 30,6 | 62,1 | 65,4 |
| | LPS | 53,4 | 56,6 | 59,1 |

Erklärung:
IMM-2414 = Lösung des Alkaloid-Standardgemisches in Medium
SN-2414 = Überstand der 7 Tage mit dem Alkaloid-Standardgemisch in Medium stimulierten Endothelzellenkultur, verdünnt mit gleichem Medium im angegebenen Verhältnis.
LPS = Lipopolysaccharid

In einer weiteren Untersuchung wurden die Überstände von mit IMM-2414 7 Tage stimulierten HUVEC-Endothelzellkulturen (SN-2414) und nicht stimulierten HUVEC-Endothelzellkulturen (SN-Medium) in verschiedenen Konzentrationen zu normalen, humanen T-Lymphozytenkulturen gegeben. Die Beeinflussung der Proliferation der Lymphozyten wurde wiederum durch Messung des [$^3$H]Thymidin-Einbaus bestimmt. Die Ergebnisse sind in Tabelle 10 wiedergegeben.

Tabelle 10 : Proliferation (cpm nach [$^3$H]Thymidin-Einbau) von humanen normalen T-Lymphozyten in Medium HAM F12

| Stimulans (Verdünnung) | T-Lymphozyten |
|---|---|
| Medium | 884 |
| IMM-2414 (2 μM) | 885 |
| und unter dem Einfluß von HUVEC-Endothelzell-Überständen | |
| SN-Medium (1:4) | 1339 |

(fortgesetzt)

| | |
|---|---|
| und unter dem Einfluß von HUVEC-Endothelzell-Überständen | |
| SN-2414 (1:4) | 1887 |
| SN-Medium (1:8) | 1106 |
| SN-2414 (1:8) | 1509 |
| SN-Medium (1:16) | 913 |
| SN-2414 (1:16) | 1279 |

Erklärung :
Medium = HAM F12, komplettiert wie vorstehend angegeben
IMM-2414 = Lösung des Alkaloid-Standardgemisches in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
Die Werte stellen Ergebnisse von Einzelexperimenten dar.

Man erkennt, daß die Alkaloide allein keinen Einfluß im Vergleich zum Medium ausüben. Die Überstände der nicht stimulierten Endothelzellkulturen (SN-Medium) führen schon zu einer Steigerung der Proliferation, die Überstände von mit IMM-2414 stimulierten HUVEC-Endothelzellkulturen (SN-2414) verstärken diese Steigerung noch deutlich. Die Dosisabhängigkeit des Effekts ist deutlich erkennbar. Die mit HUVEC-Kulturüberständen erzielten Wirkungen sind etwas schwächer, aber ebenfalls signifikant.

Eine Freisetzung des Wachstumsfaktors wird durch die Isomerengruppen der pentazyklischen Alkaloide Pteropodin und Mitraphyllin bewirkt (IMM-2414, IMM-2417 und IMM-2435), nicht aber durch die Isomerengruppe des tetrazyklischen Rhynchophyllins (IMM-2418), wie aus der vergleichenden Tabelle 11 ersichtlich ist. Auch bei diesen Untersuchungen wurden Überstände von mit IMM-2414, IMM-2417 oder IMM-2418 7 Tage stimulierten Endothelzellkulturen (SN-2414, SN-2417, SN-2418) und nicht stimulierten Endothelzellkulturen (SN-Medium) in verschiedenen Konzentrationen zu den Zellkulturen gegeben. Die Beeinflussung der Proliferation wurde durch Messung des [$^3$H]Thymidin-Einbaus bestimmt.

Tabelle 11 : Proliferation (cpm nach [$^3$H]Thymidin-Einbau) von Jurkat-Zellen (ATCC E6.1) und normalen humanen B-Lymphozyten in Medium RPMI-1640

| Stimulans (Verdünnung) | Jurkat E6.1 [a] | Stimulans (Verdünnung) | B-Lymphozyten [b] |
|---|---|---|---|
| Medium | 32737 | Medium | 632 |
| IMM-2414 (1 μM) | 35688 | IMM-2414 (1 μM) | 560 |
| IMM-2417 (1 μM) | 33700 | IMM-2417 (1 μM) | 606 |
| IMM-2418 (1 μM) | 31440 | IMM-2418 (1 μM) | 501 |
| und unter dem Einfluß von EA.hy926-Zellüberständen: | | | |
| SN-Medium (1:2) | 21673 | SN-Medium (1:8) | 1639 |
| SN-2414 (1:2) | 4282 | SN-2414 (1:8) | 2082 |
| SN-2417 (1:2) | 3953 | SN-2417 (1:8) | 2183 |
| SN-2418 (1:2) | 15724 | SN-2418 (1:8) | 1908 |
| SN-Medium (1:4) | 25288 | SN-Medium (1:16) | 1306 |
| SN-2414 (1:4) | 7163 | SN-2414 (1:16) | 1617 |
| SN-2417 (1:4) | 6068 | SN-2417 (1:16) | 2289 |
| SN-2418 (1:4) | 26132 | SN-2418 (1:16) | 1474 |
| SN-2414 (1:8) | 8044 | SN-2414 (1:32) | 1505 |
| SN-2417 (1:8) | 11783 | SN-2417 (1:32) | 2258 |

(fortgesetzt)

| | | | |
|---|---|---|---|
| und unter dem Einfluß von EA.hy926-Zellüberständen: | | | |
| SN-2418 (1:8) | 30190 | SN-2418 (1:32) | 1437 |

Erklärung :
Medium = RPMI-1640, komplettiert wie vorstehend angegeben.
IMM-2414 = Lösung des Alkaloid-Standardgemisches in Medium
IMM-2417 = Lösung des Isomerengemisches des Mitraphyllins in Medium
IMM-2418 = Lösung des Isomerengemisches des Rhynchophyllins in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
SN-2417 = Überstand der 7 Tage mit dem Isomerengemisch des Mitraphyllins in Medium stimulierten Endothelzellenkultur, verdünnt mit gleichem Medium im angegebenen Verhältnis.
SN-2418 = Überstand der 7 Tage mit dem Isomerengemisch des Rhynchophyllins in Medium stimulierten Endothelzellenkultur, verdünnt mit gleichem Medium im angegebenen Verhältnis.
[a] Die Werte stellen Mittelwerte von mindestens 3 parallelen Experimenten dar.
[b] Ergebnisse von Einzelexperimenten.

Die Alkaloide allein üben keinen Einfluß im Vergleich zum Medium aus. Die Überstände der nicht stimulierten Endothelzellkulturen (SN-Medium) führen im Fall der Jurkat-Zellen (Fig. 9) schon zu einer Hemmung, im Fall der B-Lymphozyten (Fig. 10) zu einer Steigerung der Proliferation, die Überstände von mit IMM-2414 oder IMM-2417 stimulierten Endothelzellkulturen (SN-2414, SN-2417) verstärken diese Effekte noch deutlich. Die Dosisabhängigkeit des Effekts ist deutlich erkennbar. Auffallend ist, daß Überstände von mit IMM-2417 stimulierten Endothelzellkulturen (SN-2417) auch in stärkerer Verdünnung (1:16, 1:32) zu einer Proliferationssteigerung der B-Lymphozyten führen, während die Aktivität von mit IMM-2414 stimulierten Endothelzellkulturen (SN-2414) bereits abnimmt.

Die Überstände von mit der Isomerengruppe des tetrazyklischen Rhynchophyllins (IMM-2418) stimulierten Endothelzellkulturen (SN-2418) haben keinen Einfluß im Vergleich mit Überständen nicht stimulierter Endothelzellkulturen (SN-Medium). Vielmehr konnte gezeigt werden, daß die tetrazyklischen Alkaloide sogar antagonistisch auf die Produktion und/oder Sezernierung des Wachstumsfaktors wirken (Fig. 11). Dies könnte durch ihre Eigenschaft, die $Ca^{2+}$-Ionenkanäle zu blockieren, bedingt sein. Dafür spricht auch ein zweiter Effekt: sie reduzieren dosisabhängig die vom Endothelzellfaktor bewirkte Beeinflussung der Proliferation von T-Lymphozyten (Zugabe von 1 $\mu$M IMM-2418 zu aktiven Endothelzellüberständen reduziert die cpm-Werte um 10 %, 10 $\mu$M um 20 %). Untersuchungsergebnisse finden sich in Tabelle 12. Bei den Untersuchungen wurden Überstände von mit IMM-2414 und/oder IMM-2418 7 Tage stimulierten Endothelzellkulturen (SN-2414, SN-2418, SN-2414/2418 und SN-2414/10×2418) und nicht stimulierten Endothelzellkulturen (SN-Medium) in verschiedenen Konzentrationen zu T-Lymphozyten-kulturen gegeben. Die Beeinflussung der Proliferation der Lymphozyten wurde durch Messung des [$^3$H]Thymidin-Einbaus bestimmt.

Tabelle 12: Proliferation (cpm nach [$^3$H]Thymidin-Einbau) von humanen normalen T-Lymphozyten in Medium RPMI-1640

| Stimulans (Verdünnung) | T-Lymphozyten |
|---|---|
| Medium | 598 ± 429 |
| IMM-2414 (1 $\mu$M) | 590 ± 420 |
| IMM-2418 (1 $\mu$M) | 564 ± 409 |
| IMM-2414 (1 $\mu$M)/2418 (1 $\mu$M) | 536 ± 416 |
| IMM-2414 (1 $\mu$M)/2418 (10 $\mu$M) | 602 ± 504 |
| und unter dem Einfluß von EA.hy926-Endothelzell-Überständen | |
| SN-Medium (1:4) | 1900 ± 1603 |
| SN-2414 (1:4) | 2694 ± 1662 |
| SN-2418 (1:4) | 1956 ± 1618 |
| SN-2414/2418 (1:4) | 1890 ± 1712 |
| SN-2414/10x2418 (1:4) | 1479 ± 1191 |

(fortgesetzt)

| und unter dem Einfluß von EA.hy926-Endothelzell-Überständen | |
|---|---|
| SN-Medium (1:8) | 1581 ± 1448 |
| SN-2414 (1:8) | 2144 ± 1402 |
| SN-2418 (1:8) | 1588 ± 1393 |
| SN-2414/2418 (1:8) | 1698 ± 1644 |
| SN-2414/10x2418 (1:8) | 1692 ± 1422 |

Erklärung :
Medium = RPMI-1640, komplettiert wie vorstehend angegeben.
IMM-2414 = Lösung des Alkaloid-Standardgemisches in Medium
IMM-2418 = Lösung des Isomerengemisches des Rhynchophyllins in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
SN-2418 = Überstand der 7 Tage mit dem Isomerengemisch des Rhynchophyllins in Medium stimulierten Endothelzellenkultur, verdünnt mit gleichem Medium im angegebenen Verhältnis.
SN-2414/2418 = Überstand der 7 Tage mit dem Standardgemisch und dem Isomerengemisch des Rhynchophyllins zu gleichen Teilen in Medium stimulierten Endothelzellenkultur, verdünnt mit gleichem Medium im angegebenen Verhältnis.
SN-2414/10x2418 = Überstand der 7 Tage mit dem Standardgemisch und dem Isomerengemisch des Rhynchophyllins (1:10) in Medium stimulierten Endothelzellenkultur, verdünnt mit gleichem Medium im angegebenen Verhältnis.
Die Werte stellen den Mittelwert ± Standardabweichung von 7 vergleichbaren Experimenten dar.

Man erkennt, daß die Alkaloide allein keinen Einfluß im Vergleich zum Medium ausüben. Die Überstände der nicht stimulierten Endothelzellkulturen (SN-Medium) führen schon zu einer Steigerung der Proliferation, die Überstände von mit IMM-2414 stimulierten Endothelzellkulturen (SN-2414) verstärken diese Steigerung noch deutlich. Die Überstände von mit IMM-2418 stimulierten Endothelzellkulturen (SN-2418) haben keinen Einfluß im Vergleich mit Überständen nicht stimulierter Endothelzellkulturen (SN-Medium). Die Überstände von mit IMM-2414 und IMM-2418 stimulierten Endothelzellkulturen (SN-2414/2418) haben ebenfalls keinen Einfluß im Vergleich mit Überständen nicht stimulierter Endothelzellkulturen (SN-Medium). IMM-2418 hebt also die Wirkung von IMM-2414 auf. In der höchsten getesteten Konzentration (Verdünnung 1:4) führt die Stimulation mit der 10-fachen Konzentration von IMM-2418 (SN-2414/10x2418) bereits zu einer leichten Hemmung der Proliferation im Vergleich mit Überständen nicht stimulierter Endothelzellkulturen (SN-Medium).

Untersuchungen zur Abhängigkeit der Freisetzung und / oder Wirksamkeit des Wachstumsfaktors von der Konzentration der tetrazyklischen Oxindolalkaloide in einer Gesamtoxindolalkaloidfraktion ergaben, daß mit steigendem Anteil der tetrazyklischen Oxindolalkaloide die Freisetzung und auch die Wirkung des Wachstumsfaktors abnimmt. Einer Lösung mit einer konstanten Konzentration der pentazyklischen Oxindolalkaloide von ≈1μM wurden tetrazyklische Oxindolalkaloide in den Konzentrationen 0.01, 0.1 und 1μM zugemischt. Mit diesen Mischungen wurden EA.hy 926-Endothelzellen über 7 Tage inkubiert. Die Überstände der Endothelzellkulturen wurden anhand des [$^3$H]Thymidin-Einbaus an Raji- und Jurkat-Zellen auf ihre Aktivität getestet. In Tabelle 13 sind die Mittelwerte der Profilerationshemmung durch die Mischungsüberstände aus 3 Verdünnungsstufen (1:4, 1:8 und 1:16) im Vergleich zur Positivkontrolle (reine pentazyklische Alkaloide) in % angegeben.

Tabelle 13: Proliferationshemmende Aktivität von EA.hy926-Endothelzell-Überständen (SN) aus Kulturen, die mit IMM-2414 bzw. IMM-2417 in Kombination mit IMM-2418 inkubiert wurden, im Vergleich zur Positivkontrolle (%)

| | Jurkat E6.1 | Raji CCL86 |
|---|---|---|
| SN-2414 (1 μM) | 100 | 100 |
| SN-[2414 (1 μM) + 2418 (0.01 μM)] | 58 | 74 |
| SN-[2414 (1 μM) + 2418 (0.1 μM)] | 50 | 52 |
| SN-[2414 (1 μM) + 2418 (1 μM)] | 25 | 2 |
| SN-2418 (1 μM) | -24 . | -3 |

(fortgesetzt)

| | Jurkat E6.1 | Raji CCL86 |
|---|---|---|
| SN-2417 (1 μM) | 100 | 100 |
| SN-[2417 (1 μM) + 2418 (0.01 μM)] | 68 | 69 |
| SN-[2417 (1 μM) + 2418 (0.1 μM)] | 54 | 45' |
| SN-[2417 (1 μM) + 2418 (1 μM)] | 22 | 24 |
| SN-2418 (1 μM) | -15 | 3 |

Erklärung:
SN-2414 = Überstand der 7 Tage mit dem Alkaloid-Standardgemisch in Medium stimulierten Endothelzellenkultur, verdünnt mit gleichem Medium im angegebenen Verhältnis.
SN-2417 = Überstand der 7 Tage mit dem Isomerengemisch des Mitraphyllins in Medium stimulierten Endothelzellenkultur, verdünnt mit gleichem Medium im angegebenen Verhältnis.
SN-2418 = Überstand der 7 Tage mit dem Isomerengemisch des Rhynchophyllins in Medium stimulierten Endothelzellenkultur, verdünnt mit gleichem Medium im angegebenen Verhältnis.

Die $EC_{50}$ (jene Konzentration an tetrazyklischen Alkaloiden, bei welcher der durch pentazyklische Alkaloide induzierte Endothetzell-Überstand nur mehr 50 % seiner profilerationshemmenden Aktivität aufweist) wurde mit 10 Vol. % tetrazyklische Alkaloide ermittelt. Wie ersichtlich, verändert sich die proliferationssenkende Wirkung des gewonnenen Überstandes auf transformierte Lymphoblasten in Abhängigkeit von der Dosierung der tetrazyklischen Oxindolalkaloide (Fig. 12).

Um sicherzustellen, daß nicht die Alkaloide allein oder im Zusammenwirken mit einem an sich vorhandenen Endothelzellfaktor die Wirkung auf Lymphozyten ausüben, wurden die wirksamen Isomerengruppen der Oxindolalkaloide allein zu Lymphozyten gegeben, und es wurden Endothelzellen ohne Alkaloide kultiviert und dem Überstand anschließend die Alkaloide zugegeben. Beide Versuchsreihen zeigten, daß die Alkaloide weder für sich allein noch in Kombination mit dem Überstand die Proliferation der Lymphozyten beeinflußten. Damit war bewiesen, daß die Gruppen der pentazyklischen Isomeren nicht direkt auf die Proliferation Einfluß nehmen, sondern daß sie Endothelzellen zur Produktion und/oder Sezernierung eines Wachstumsfaktors anregen, der die Proliferation von Lymphozyten beeinflußt.

Es wird angenommen, daß Endothelzellen einen ähnlich wirkenden Faktor auch ohne Stimulation in geringer Konzentration produzieren, weil, wie aus den Tabellen ersichtlich ist, auch die Überstände der nicht stimulierten Endothelzellkulturen (SN-Medium) die Proliferation von Lymphozyten beeinflussen, wenn auch in wenig ausgeprägter Weise. Untersuchungen mit pentazyklischen Oxindolalkaloiden in niedriger Dosierung (0.1 μM) führten nur in seltenen Fällen zu einer Produktion und/oder Sezernierung des proliferationsregelnden Faktors, wie aus Tabelle 14 ersichtlich ist. Auch hier wurden Überstände von mit IMM-2414 (c = 0.1 μM) 7 Tage stimulierten Endothelzellkulturen (SN-2414) und nicht stimulierten Endothelzellkulturen (SN-Medium) in verschiedenen Konzentrationen zu Kulturen lymphoblastoider Zellen gegeben. Die Proliferation der transformierten Zellen wurde nach 2 Tagen durch Messung des $[^3H]$Thymidin-Einbaus bestimmt.

Tabelle 14 : Proliferation (cpm nach $[^3H]$Thymidin-Einbau) von lymphoblastoiden Zell-Linien in Medium RPMI-1640

| Stimulans (Verdünnung) | Raji CCL86 | Jurkat E6.1 |
|---|---|---|
| Medium | 27617 | 42536 |
| IMM-2414 (0.1 μM) | 28708 | 42779 |
| und unter dem Einfluß von EA.hy926-Endothelzellkultur-Überständen | | |
| SN-Medium (1:2) | 38556 | 35917 |
| SN-2414 (1:2) | 36397 | 35653 |
| SN-Medium (1:4) | 36227 | 38982 |
| SN-2414 (1:4) | 28558 | 34048 |
| SN-Medium (1:8) | 30785 | 40389 |

(fortgesetzt)

| und unter dem Einfluß von EA.hy926-Endothelzellkultur-Überständen | | |
|---|---|---|
| SN-2414 (1:8) | 22661 | 39902 |

Erklärung :
Medium = RPMI-1640, komplettiert wie vorstehend angegeben.
IMM-2414 = Lösung des Alkaloid-Standardgemisches in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.

Man erkennt, daß die Alkaloide allein keinen Einfluß im Vergleich zum Medium ausüben. Die Überstände der mit IMM-2414 in niedriger Dosierung stimulierten Endothelzellkulturen (SN-2414) beeinflussen die Proliferation der Raji- und Jurkat-Zellen nur mehr schwach. Der proliferationsregelnde Faktor bindet an Interferon-β-Antiserum vom Schaf und kann an Sepharose isoliert werden.

Es ist anzunehmen, daß der Endothelzellfaktor aufgrund seiner für normale B- und T-Lymphozyten proliferationssteigernden Eigenschaft auch zur modifizierten Ausschüttung anderer Faktoren des Immunsystems führt, die ihrerseits von immunkompetenten Zellen produziert werden, wie beispielsweise Interferon-y, TNF-a, verschiedene Interleukine sowie den Granulozyten und Makrophagen stimulierenden Faktor GM-CSF. Auch wenn die Wirkungen des proliferationsregelnden Faktors durch immunologische Regelkreise auf die gegebene Immunlage abgestimmt werden, scheint es sinnvoll, diese Wirkungen auch gezielt begrenzen zu können. Die tetrazyklischen Oxindolalkaloide sind hierfür einsetzbar, da sie dosisabhängig die Wirkung des durch die pentazyklischen Isomerengruppen induzierten Faktors hemmen.

Ergänzend wurden Versuche mit den eingangs beschriebenen einfachen Derivaten der pentazyklischen Oxindolalkaloide durchgeführt. Hiefür wurden Überstände von mit IMM-2414, IMM-2413 oder IMM-2433 7 Tage stimulierten Endothelzellkulturen (SN-2414, SN-2413, SN-2433) und nicht stimulierten Endothelzellkulturen (SN-Medium) in verschiedenen Konzentrationen zu T-Lymphozytenkulturen gegeben. Die Beeinflussung der Proliferation der Lymphozyten wurde durch Messung des [$^3$H]Thymidin-Einbaus bestimmt. Es zeigte sich, daß die entsprechenden Carbonsäuren schwach und die N-Oxide kaum aktiv sind (Tabelle 15). Denkbar ist, daß diese Derivate aufgrund ihrer im Vergleich zu den ursprünglichen Alkaloiden höheren Polarität in geringerem Ausmaß in die Zellen eindringen oder an ihre Bindungspartner binden.

Tabelle 15 : Proliferation (cpm nach [$^3$H]Thymidin-Einbau) von humanen normalen T-Lymphozyten in Medium RPMI-1640

| Stimulans (Verdünnung) | T-Lymphozyten |
|---|---|
| Medium | 1724 |
| IMM-2414 (1 μM) | 1309 |
| IMM-2413 (1 μM) | 876 |
| IMM-2433 (1 μM) | 1305 |
| und unter dem Einfluß von EA.hv926-Endothelzellkultur-Überständen | |
| SN-Medium (1:4) | 2366 |
| SN-2414 (1:4) | 4864 |
| SN-2413 (1:4) | 2717 |
| SN-2433 (1:4) | 2418 |
| SN-Medium (1:8) | 2079 |
| SN-2414 (1:8) | 3103 |
| SN-2413 (1:8) | 2850 |
| SN-2433 (1:8) | 2327 |
| SN-Medium (1:16) | 2062 |
| SN-2414 (1:16) | 2493 |

(fortgesetzt)

| | |
|---|---|
| und unter dem Einfluß von EA.hv926-Endothelzellkultur-Überständen | |
| SN-2413 (1:16) | 2049 |
| SN-2433 (1:16) | 1994 |

Erklärung :
Medium = RPMI-1640, komplettiert wie vorstehend angegeben.
IMM-2414 = Lösung des Alkaloid-Standardgemisches in Medium
IMM-2413 = Lösung von Carbonsäuren des Alkaloid-Standardgemisches in Medium
IMM-2433 = Lösung von N-Oxiden des Alkaloid-Standardgemisches in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.
SN-2413 = Überstand der 7 Tage mit den Alkaloid-Carbonsäuren in Medium stimulierten Endothelzellenkultur, verdünnt mit gleichem Medium im angegebenen Verhältnis.
SN-2433 = Überstand der 7 Tage mit den Alkaloid-N-Oxiden in Medium stimulierten Endothelzellenkultur, verdünnt mit gleichem Medium im angegebenen Verhältnis.
Die Werte stellen Ergebnisse von Einzelexperimenten dar.

Man erkennt, daß die Alkaloide allein keinen Einfluß im Vergleich zum Medium ausüben. Die Überstände der nicht stimulierten Endothelzellkulturen (SN-Medium) führen schon zu einer Steigerung der Proliferation, die Überstände von mit IMM-2414 stimulierten Endothelzellkulturen (SN-2414) verstärken diese Steigerung noch deutlich. Die Überstände von mit IMM-2413 stimulierten Endothelzellkulturen (SN-2413) haben nur schwachen, die Überstände von mit IMM-2433 stimulierten Endothelzellkulturen (SN-2433) kaum Einfluß im Vergleich mit Überständen nicht stimulierter Endothelzellkulturen (SN-Medium).

Aus diesen Untersuchungen und Überlegungen geht hervor, daß die Mischung der den Endothelzellen zugeführten Oxindolalkaloide im Sinne einer zeitlich definierten Wirkung nicht beliebig gewählt oder dem Zufall überlassen werden kann. Besonderes Augenmerk wurde auf die proliferationshemmende Wirkung des durch pentazyklische Oxindolalkaloide induzierten Faktors auf leukämische und virustransformierte Lymphozyten gelegt. Die Proliferationshemmung erreicht mit bis zu 87 % ein Ausmaß, das zytotoxische Wirkungen des Faktors erwarten ließ. Mit Hilfe der Trypanblau-Färbung wurde daher die Viabilität dieser Zellen untersucht. Trotz starker Proliferationshemmung wurde keine Änderung der Viabilität festgestellt, wie die Tabelle 16 zeigt. Die Wirkung des Faktors ist daher nicht auf zytotoxische Effekte zurückzuführen.

Tabelle 16 : Viabilität (in %) der Raji- und Jurkat-Zellen in Medium RPMI-1640

| Stimulans (Verdünnung) | Raji CCL86 | | Jurkat E6.1 | |
|---|---|---|---|---|
| | 1. Tag | 2. Tag | 1. Tag | 2. Tag |
| Medium | 93.5 | 95.1 | 95.8 | 93.7 |
| 1 μM IMM-2414 | 96.8 | 90.1 | 95.2 | 95.8 |
| und unter dem Einfluß von EA.hy926-Endothelzellkultur-Überständen | | | | |
| SN-Medium (1:2) | 95.4 | 95.3 | 96.4 | 92.0 |
| SN-2414 (1:2) | 98.4 | 92.2 | 94.4 | 92.2 |

Erklärung :
Medium = RPMI-1640, komplettiert wie vorstehend angegeben.
IMM-2414 = Lösung des Alkaloid-Standardgemisches in Medium
SN-Medium = Überstand der Endothelzellenkultur in Medium, verdünnt mit gleichem Medium im angegebenen Verhältnis
SN-2414 = Überstand der 7 Tage mit dem Alkaloid-Standardgemisch in Medium stimulierten Endothelzellenkultur, verdünnt mit gleichem Medium im angegebenen Verhältnis.

Um zu zeigen, daß die Hemmung der Proliferation der transformierten lymphoblastoiden Zell-Linien Raji CCL86 und Jurkat E6.1 nicht auf zytotoxische Effekte zurückzuführen ist, wurde die Viabilität der untersuchten Zellen mittels

Trypanblau-Färbbarkeit am 1. und 2. Tag der Stimulation bestimmt. Man erkennt, daß die Zellen, unbeeinflußt von den Stimulantien, in allen Fällen eine Viabilität über 90 % aufweisen.

Zur weiteren Charakterisierung des Wachstumsfaktors wurde der Überstand von mit IMM-2414 kultivierten Endothelzellkulturen durch verschiedene Molekulargewichtsfilter filtriert und die Fraktionen auf ihre proliferationsregelnde Aktivität überprüft. Fraktionen mit einem Molekulargewicht unter 30,000 erwiesen sich als inaktiv, während Fraktionen über 30,000 Aktivität zeigten, allerdings in geringerem Ausmaß als die nicht filtrierte Kontrolle. Daraus kann geschlossen werden, daß der durch pentazyklische Oxindolalkaloide induzierte proliferationsregelnde Faktor entweder ein Molekulargewicht um 30,000 besitzt und nur teilweise das Filter passierte oder daß kleinere Komponenten, die für die volle Aktivität notwendig sind, das Filter passierten.

## Diskussion

Pflanzen, die Oxindolalkaloide synthetisieren, neigen dazu, abhängig von Umwelteinflüssen entweder tetrazyklische oder pentazyklische Oxindolalkaloide auszubilden. Die Wirkungen dieser beiden Alkaloidtypen sind grundsätzlich verschieden, so daß eine Unterscheidung der beiden Pflanzenmodifikationen entscheidend für die Herstellung von Arzneimitteln ist.

Oxindolalkaloide isomerisieren in wässerigen Lösungsmitteln. Abhängig vom pH-Wert und von der Gegenwart mit Wasser nicht mischbarer Lösungsmittel bilden sie unterschiedliche Isomerenmischungen. Die Isomerisierung bis zum Gleichgewicht erfolgt bei 37 °C in reinem Wasser innerhalb von rund 24 Stunden, in Gegenwart von Lipiden oder Bindungspartnern verlängert sich diese Zeit. Die physiologisch sich einstellenden Isomerengemische oder einzelne Isomere setzen ab einer bestimmten, von der Aktivität der Zellen abhängigen Minimalkonzentration aus Endothelzellen einen Faktor frei, der die Proliferation von Lymphozyten regelnd beeinflußt. Das Ausmaß der Wirkung dieses Faktors wird weitgehend von den Regelkreisen des Immunsystems bestimmt und kann durch Veränderung der Konzentration der pentazyklischen Oxindolalkaloide nicht beeinflußt werden. Die tetrazyklischen Oxindolalkaloide wirken dosisabhängig als Antagonisten auf die Produktion und/oder Sezernierung dieses Faktors und sind zudem in der Lage, die Wirkung des Faktors selbst abzuschwächen. Sie sind daher geeignet, die Wirkung der pentazyklischen Oxindolalkaloide zu steuern. Die Wirkung des Faktors ist eine Steigerung der Proliferation ruhender und schwach aktivierter Lymphozyten, sowie eine Hemmung der Proliferation von Lymphoblasten, leukämischen Lymphozyten und virustransformierten Lymphozyten. Die Viabilität der in der Proliferation gehemmten Zellen bleibt dabei unberührt.

Das Molekulargewicht des Faktors oder seiner Komponenten liegt zwischen 20,000 und 40,000. In lebenden Organismen ist die Ausscheidung der Oxindolalkaloide zu berücksichtigen. Die unterschiedliche Wasser- und Lipidlöslichkeit der einzelnen Oxindolalkaloide ist bei der Zubereitung einer Darreichungsform zu berücksichtigen. Wenn die Ausschüttung des Endothelzellfaktors in gezieltem Ausmaß induziert werden soll, müssen die Isomerengruppen in einem Mischungsverhältnis verabreicht werden, das möglichst der therapeutischen Notwendigkeit angepaßt ist. Die Dosierung muß so hoch sein, daß die Konzentration der wirksamen Isomeren im Inneren der Endothelzellen nicht unter die für die Freisetzung des Wachstumsfaktors notwendige Mindestkonzentration absinkt. Aufgrund der Eigenschaft dieses Faktors, die Proliferation normaler Lymphozyten zu fördern, können andere Faktoren, die ihrerseits von den Lymphozyten produziert werden, verstärkt zur Wirkung gebracht oder gewonnen werden.

Zum Studium der Bioäquivalenz von reinen pentazyklischen Alkaloidfraktionen und rein pentazyklische Oxindolalkaloide enthaltenden Extrakten aus der Wurzel von Uncaria tomentosa wurde aus dieser Droge ein HCl-saurer Extrakt hergestellt und Endothelzellen in einer Menge zugegeben, die zur gleichen Konzentration der Alkaloide wie bei den reinen Alkaloidfraktionen führte. Die Wirkung der so gewonnenen Endothelzellüberstände auf die Proliferation von Jurkat- und Raji-Zellen hatte etwa 80% der Wirkung der reinen Alkaloidfraktion IMM-2414.

Die Verwendung des pentazyklischen Oxindolalkaloids Isopteropodin und seiner Isomerengruppe zur Steigerung der Phagozytoseleistung der Granulozyten ist, wie einleitend erwähnt, patentiert worden. Dabei handelt es sich um eine Stimulierung von Zellen des Immunsystems. Die Verwendung der pentazyklischen Oxindolalkaloide zur Freisetzung eines Lymphozyten-Wachstumsfaktors aus Endothelzellen, die nicht dem Immunsystem gehören, ist prinzipiell neu. Auch dieser Faktor, der die Proliferation der Lymphozyten in Richtung ihres physiologischen Gleichgewichts verändert, ist bisher nicht beschrieben worden. Seine Wirkung ist nicht eine Stimulierung, sondern eine Regelung immunologischer Vorgänge.

Erläuterung der Zeichnungsfiguren:

Fig. 1 : Qualitative Unterscheidung der beiden Modifikanten von Uncaria tomentosa mit Dünnschichtchromatographie, wobei die Spalten 1 und 2 die Ergebnisse von Lösungen aus Uncaria tomentosa mod.pent., die Spalten 3 und 4 die Ergebnisse von Lösungen aus Uncaria tomentosa mod.tetr. und die Spalten 6 und 7 die Ergebnisse von Mischlösungen zeigen. Die Spalte 5 zeigt das Ergebnis der Referenzlösung.

Fig. 2 : Trennung von pentazyklischen und tetrazyklischen Oxindolalkaloiden mittels HPLC.

Fig. 3 : Schematische Darstellung der Isomerisierungsgleichgewichte $K_{aq}$, $K_{org}$ und Verteilungsgleichgewichte

$K_{org/aq}$ zweier Isomere (1) und (2) im Zwei-Phasen-System.

$$\text{Es gilt } K_{org}(1 \rightarrow 2) = \frac{K_{org/aq}(2)}{K_{org/aq}(1)} K_{aq}(1 \rightarrow 2).$$

Fig. 4 : pH-Abhängigkeit der Gleichgewichtskonzentrationen (in %) im System Octanol-Wasser am Beispiel von Mitraphyllin und Isomitraphyllin.
Fig. 5 : Steigerung der Proliferation von normalen humanen B-Lymphozyten, stimuliert mit Überständen von mit je 1μM IMM-2414, 2417, 2418 kultivierten EA.hy926-Zellen (Einzelexperiment).
Fig. 6 : Hemmung der Proliferation von Jurkat-Zellen (ATCC E6.1), stimuliert mit Überständen von mit je 1μM IMM-2414, 2417, 2418 kultivierten EA.hy926-Zellen (Mittelwerte von mindestens 3 parallelen Experimenten).
Fig. 7 : Hemmung der Proliferation (cpm nach [$^3$H]Thymidin-Einbau) von hochaktivierten humanen B- und T-Lymphozyten (Lymphoblasten) durch EA.hy926-Endothelzellkultur-Überstände (SN).
Fig. 8 : Hemmung der Proliferation (cpm nach [$^3$H]Thymidin-Einbau) von humanen hoch aktivierten T-Lymphozyten (Lymphoblasten) unter dem Einfluß von IMM-2414, IMM-2417 oder IMM-2435 allein und EA.hy926-Endothelzell-Überständen (SN) aus Kulturen, die mit IMM-2414, IMM-2417 oder IMM-2435 inkubiert wurden
Fig. 9: Proliferation (cpm nach [$^3$H]Thymidin-Einbau) von Jurkat-Zellen (ATCC E6.1), stimuliert mit Überständen von mit je 1μM IMM-2414, 2417, 2418 kultivierten EA.hy926-Zellen
Fig. 10 : Proliferation (cpm nach [$^3$H]Thymidin-Einbau) von normalen humanen B-Lymphozyten, stimuliert mit Überständen von mit je 1μM IMM-2414, 2417, 2418 kultivierten EA.hy926-Zellen
Fig. 11 : Proliferation (cpm nach [$^3$H]Thymidin-Einbau) von humanen normalen T-Lymphozyten unter dem Einfluß von IMM-2414 und/oder IMM-2418 allein und EA.hy926-Endothelzell-Überständen (SN) aus Kulturen, die mit IMM-2414 und/oder IMM-2418 inkubiert wurden
Fig. 12: Antagonistische Wirkung von tetrazyklischen Oxindolalkaloiden auf die durch pentazyklische Oxindolalkaloide hervorgerufenen biologischen Effekte auf Raji- und Jurkat-Zellen.

**Ausführungsbeispiele**

1. Normale humane Nabelschnur-Endothelzellen (HUVEC, ATCC CRL-1730) werden 7 Tage lang bei 37°C in HAM F12-Nährmedium kultiviert, das mit 10 Vol.% fötalem Kälberserum, 60 μg/ml EndothelialCellGrowthStimulant und 100 μg/ml Heparin komplettiert wurde und pentazyklische Oxindolalkaloide (c = 0.4 mg/l) enthält. Anschließend wird der Überstand entnommen, sterilfiltriert, mit dem gleichen Nährmedium im Verhältnis 1:4 verdünnt und zu Kulturen von normalen humanen T-Lymphozyten gegeben. Die Gegenwart des aus den Endothelzellen freigesetzten Faktors führt innerhalb von 5 Tagen zu einer Steigerung der Proliferation der Lymphozyten um 110 %.

2. Transformierte EA.hy926-Endothelzellen werden 7 Tage lang bei 37°C in RPMI-1640-Nährmedium kultiviert, das mit 10 Vol.% fötalem Kälberserum, 2 mM Glutamin, 50 Einheiten/ml Penicillin G und 50 μg/ml Streptomycin komplettiert wurde und pentazyklische Oxindolalkaloide (c = 0.4 mg/l) enthält. Anschließend wird der Überstand entnommen, sterilfiltriert, mit dem gleichen Nährmedium im Verhältnis 1:4 verdünnt und zu Kulturen von normalen humanen B-Lymphozyten gegeben. Die Gegenwart des aus den Endothelzellen freigesetzten Faktors führt innerhalb von 5 Tagen zu einer Steigerung der Proliferation der Lymphozyten um 330 %.

3. Transformierte EA.hy926-Endothelzellen werden 7 Tage lang bei 37°C in RPMI-1640-Nährmedium kultiviert, das mit 10 Vol.% fötalem Kälberserum, 2 mM Glutamin, 50 Einheiten/ml Penicillin G und 50 μg/ml Streptomycin komplettiert wurde und Pteropodin und Isopteropodin (c = 0.4 mg/l) enthält. Anschließend wird der Überstand entnommen, sterilfiltriert, mit dem gleichen Nährmedium im Verhältnis 1:2 verdünnt und zu Kulturen von leukämischen Jurkat-Zellen (ATCC E6.1) gegeben. Die Gegenwart des aus den Endothelzellen freigesetzten Faktors führt innerhalb von 2 Tagen zu einer Abschwächung der Proliferation um 80 %.

4. Transformierte EA.hy926-Endothelzellen werden 7 Tage lang bei 37°C in RPMI-1640-Nährmedium kultiviert, das mit 10 Vol.% fötalem Kälberserum, 2 mM Glutamin, 50 Einheiten/ml Penicillin G und 50 μg/ml Streptomycin komplettiert wurde und Mitraphyllin und Isomitraphyllin (c = 0.4 mg/l) enthält. Der Überstand wird entnommen, sterilfiltriert, mit dem gleichen Nährmedium im Verhältnis 1:4 verdünnt und zu Kulturen von hoch aktivierten humanen T-Lymphozyten (Lymphoblasten) gegeben. Die Gegenwart des aus den Endothelzellen freigesetzten Faktors führt innerhalb von 2 Tagen zu einer Abschwächung der Proliferation der Lymphozyten um 40 %.

5. Transformierte EA.hy926-Endothelzellen werden 7 Tage lang bei 37°C in RPMI-1640-Nährmedium kultiviert, das mit 10 Vol.% fötalem Kälberserum, 2 mM Glutamin, 50 Einheiten/ml Penicillin G und 50 μg/ml Streptomycin komplettiert wurde und Mitraphyllin und Isomitraphyllin (c = 0.4 mg/l) beziehungsweise Pteropodin und Isopteropodin (c = 0.4 mg/l) enthalten. Nach 7 Tagen werden die Überstände entnommen, sterilfiltriert und in verschiedenen Verdünnungen mit dem gleichen Nährmedium (1:8, 1:16, 1:32) zu Kulturen von normalen humanen B-Lymphozyten gegeben. Der durch Stimulation mit der Mitraphyllin-Gruppe gewonnene Überstand fördert zumindest bis zur Verdünnung 1:32 die Proliferation der Lymphozyten um 260 %, der durch Stimulation mit der Pteropodin-Gruppe gewonnene Überstand zeigt eine dosierungsabhängige Abnahme der proliferationssteigernden Wirkung (250 %, 170 % und 150 %).

6. Normale humane Nabelschnur-Endothelzellen (HUVEC, ATCC CRL-1730) werden 7 Tage lang bei 37°C in HAM F12-Nährmedium kultiviert, das mit 10 Vol.% fötalem Kälberserum, 60 μg/ml EndothelialCellGrowthStimulant und 100 μg/ml Heparin komplettiert wurde und pentazyklische Oxindolalkaloide (c = 0.4 mg/l) enthält. Anschließend wird der Überstand entnommen und sterilfiltriert. Pro ml Überstand werden 50 μl Interferon-β-Antiserum (vom Schaf) in sterilem Wasser (c = 19000 U/ml) zugegeben, gut durchmischt und 1 Stunde bei 4°C inkubiert. Dann gibt man 25 μl 10 % Protein-A-Sepharose-Suspension zu, inkubiert 30 Minuten bei 4°C und zentrifugiert mit 20000 N. Aus dem Sediment eluiert man den Faktor-Antiserum-Komplex mit einer 0.1 M Lösung von Gly-cin HCl bei pH 2.6.

7. Extrakte aus der Wurzel von Uncaria tomentosa werden mit Hochleistungsflüssigkeitschromatografie (HPLC) auf Abwesenheit von tetrazyklischen Oxindolalkaloiden geprüft. Es wird eine RP-18 (5 μm)-Säule (125 × 4 mm) verwendet und mit einem Gemisch aus Acetonitril und 0.01 M Phosphatpuffer pH 7 (40:60) bei einem Fluß von 1.3 ml/min und einer Temperatur von 52°C chromatografiert. Dadurch erreicht man eine zuverlässige Trennung von tetrazyklischen und pentazyklischen Oxindol-Alkaloiden (Fig. 2). Die Detektion erfolgt bei einer Wellenlänge von 247 nm. Nur Extrakte, die ausschließlich pentazyklische Oxindol-Alkaloide enthalten, werden zur weiteren Verwendung herangezogen. Solche Extrakte werden zu galenischen Formen verarbeitet, beispielsweise zur Abfüllung in Hartgelatine. Kapseln durch Vermischung des Extraktes (20 mg/Kapsel) mit Ascorbinsäure (200 mg/Kapsel) und Lactose (130 mg/Kapsel), oder zur Herstellung von Tropfen durch Lösung der Extrakte (600 mg/100 g Tropfenlösung) in Ethanol (8,15 g/100 g Tropfenlösung) und Wasser (90,89 g/100 g Tropfenlösung). An Patienten mit krankheitsbedingten oder therapiebedingten Fehlregulationen des Immunsystems werden diese Präparate verabreicht.

8. Einem Tumorpatienten, dessen Lymphozytenzahl durch Chemotherapie (Taxol®) vermindert ist (relative und absolute Lymphopenie, Lymphozytenanteil 18 % der Leukozyten, 1.1 G/l), wird ergänzend zu dieser Therapie Extrakt aus der Wurzel von *Uncaria tomentosa mod. pent.* verabreicht, in der Dosierung entsprechend 0.6 mg pentazyklische Oxindolalkaloide täglich. Trotz fortgesetzter Chemotherapie steigen innerhalb eines Monats die Lymphozytenzahlen signifikant an (weißes Blutbild ohne pathologischen Befund, Lymphozytenanteil 21 % der Leukozyten, 1.4 G/l).

Der folgende Punkt zeigt nicht ein Ausführungsbeispiel der Erfindung:

Ein Extrakt aus der Wurzel von *Uncaria tomentosa mod. pent.*, der etwa 1 Gew.% pentazyklische Oxindolalkaloide enthält, wird in der Dosierung von 1 g/kg Körpergewicht täglich an gesunde Ratten oral verabreicht. Innerhalb von 28 Tagen bildet sich eine signifikante, relative und absolute Lymphozytose aus (bei 5 männlichen Ratten von 86.8 % auf 90.4 %, von 8.4 G/l auf 8.5 G/l; bei 5 weiblichen Ratten von 83.4 % auf 88.4 %, von 4.9 G/l auf 5.7 G/l).

## Patentansprüche

1. Verfahren zur Freisetzung eines Lymphozytenwachstumsfaktors aus in einem Nährmedium kultivierten Endothelzellen in den Überstand der Endothelzellkultur, indem zumindest ein pentazyklisches Oxindolalkaloid in das Nährmedium zugeführt wird.

2. Verfahren nach Anspruch 1, **dadurch gekenntzeichnet, daß** die durch pentazyklische Oxindolalkaloide erzielbare Freisetzung des Lymphozytenwachstumsfaktors durch Zugabe zumindest eines antagonistisch wirkenden, tetrazyklischen Oxindolalkaloids in das Nährmedium begrenzt wird.

3. Verfahren zur Herstellung eines einen Lymphozytenwachstumsfaktor enthaltenden Präparates für die Regulierung

der Proliferation von Lymphozyten, wobei eine Endothelzellkultur zumindest mit einem pentazyklischen Oxindolalkaloid versetzt wird, worauf die Endothelzellen den Wachstumsfaktor in den Überstand der Endothelzellkultur freisetzen, und der den Wachstumsfaktor enthaltende Überstand von der Endothelzellkultur getrennt wird.

**4.** Verwendung des Überstandes einer Endothelzellkultur, die mit zumindest einem pentazyklischen Oxindolalkaloid versetzt wurde, zur Herstellung eines Mittels zur Förderung der Proliferation ruhender und schwach aktivierter Lymphozyten sowie zur Hemmung der Proliferation hochaktivierter und transformierter Lymphozyten.

**5.** Verwendung des Überstandes einer Endothelzellkultur, die mit zumindest einem pentazyklischen Oxindolalkaloid versetzt wurde, zur Herstellung eines Arzneimittels.

**6.** Stoffgemisch enthaltend zumindest ein pentazyklisches Oxindolaikaloid als Mittel, das Endothelzellen zur Freisetzung eines Lymphozytenwachstumsfaktors stimuliert zur Regelung des Lymphozytenwachstums bei krankheitsbedingten oder therapiebedingten Fehlregulationen des Immunsystems.

## Claims

**1.** A method of releasing a lymphocyte growth-factor from endothelial cells cultivated in a nutrient medium into the supernatant of the endothelial cell culture by addition of at least one pentacyclic oxindole alkaloid into the nutrient medium.

**2.** The method according to claim 1, **characterized in that** the extent of release of the lymphocyte growth-factor by the pentacyclic oxindole alkaloid is limited by the addition of at least one antagonistically active tetracyclic oxindole alkaloid into the nutrient medium.

**3.** A method of producing a preparation containing a lymphocyte growth-factor for a regulation of proliferation of lymphocytes, wherein to an endothelial cell culture is added at least one pentacyclic oxindole alkaloid, whereupon the endothelial cells release the growth-factor into the supernatant of the endothelial cell culture, and wherein the supernatant containing the growth-factor is separated from the endothelial cell culture.

**4.** Use of a supernatant of an endothelial cell culture to which at least one pentacyclic oxindole alkaloid has been added, for producing a substance enhancing proliferation of resting and weakly activated lymphocytes and inhibiting proliferation of highly activated and transformed lymphocytes.

**5.** Use of a supernatant of an endothelial cell culture to which at least one pentacyclic oxindole alkaloid has been added, for producing a medicine.

**6.** Mixture of materials containing at least one pentacyclic oxindole alkaloid, as an agent that stimulates endothelial cells to release a lymphocyte growth-factor, to regulate the growth of lymphocytes when regulations of the immunologic system are deficient due to disease or therapy.

## Revendications

**1.** Procédé de libération d'un facteur de croissance de lymphocytes à partir de cellules endothéliales cultivées dans un milieu de culture, dans le liquide surnageant d'une culture de cellules endothéliales, dans lequel au moins un alcaloïde pentacyclique de l'oxindole est introduit dans le milieu de culture.

**2.** Procédé selon la revendication 1, **caractérisé en ce que** la libération du facteur de croissance de lymphocytes réalisable par des alcaloïdes pentacycliques de l'oxindole est limitée par l'addition dans le milieu de culture d'au moins un alcaloïde tétracyclique de l'oxindole à action antagoniste.

**3.** Procédé pour la fabrication d'une préparation pour la régulation de la prolifération des lymphocytes, contenant un facteur de croissance de lymphocytes, dans lequel on additionne au moins un alcaloïde pentacyclique de l'oxindole à une culture de cellules endothéliales, après quoi les cellules endothéliales libèrent le facteur de croissance dans le liquide surnageant du milieu de culture de cellules endothéliales, et le liquide surnageant contenant le facteur de croissance est séparé de la culture de cellules endothéliales.

4. Utilisation du liquide surnageant d'une culture de cellules endothéliales, qui a été additionnée d'au moins un alcaloïde pentacyclique de l'oxindole, pour la fabrication d'un produit pour la promotion de la prolifération de lymphocytes au repos et faiblement activés ainsi que pour l'inhibition de la prolifération de lymphocytes hautement activés et transformées.

5. Utilisation du liquide surnageant d'une culture de cellules endothéliales, qui a été additionnée d'au moins un alcaloïde pentacyclique de l'oxindole, pour la fabrication d'un médicament.

6. Mélangé de substances, contenant au moins un alcaloïde pentacyclique de l'oxindole comme moyen, qui stimule des cellules endothéliales pour libérer un facteur de croissance de lymphocytes, pour la régulation de la croissance de lymphocytes en cas de défauts de régulation du système immunitaire, causés par une maladie ou causés par une thérapie.

Fig. 1

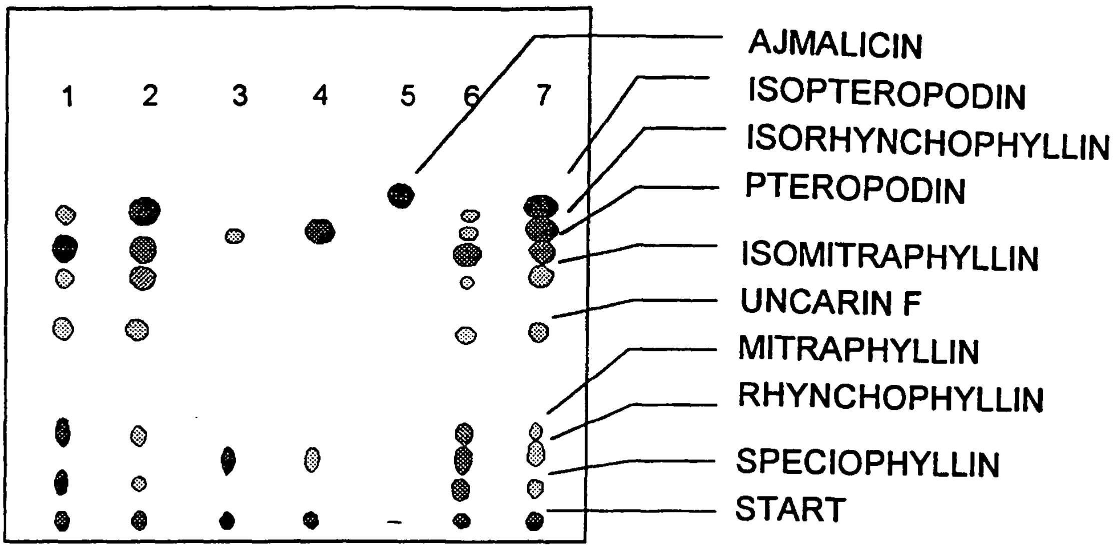
1
2
3
4
5
6
7
AJMALICIN
ISOPTEROPODIN
ISORHYNCHOPHYLLIN
PTEROPODIN
ISOMITRAPHYLLIN
UNCARIN F
MITRAPHYLLIN
RHYNCHOPHYLLIN
SPECIOPHYLLIN
START

Fig. 2

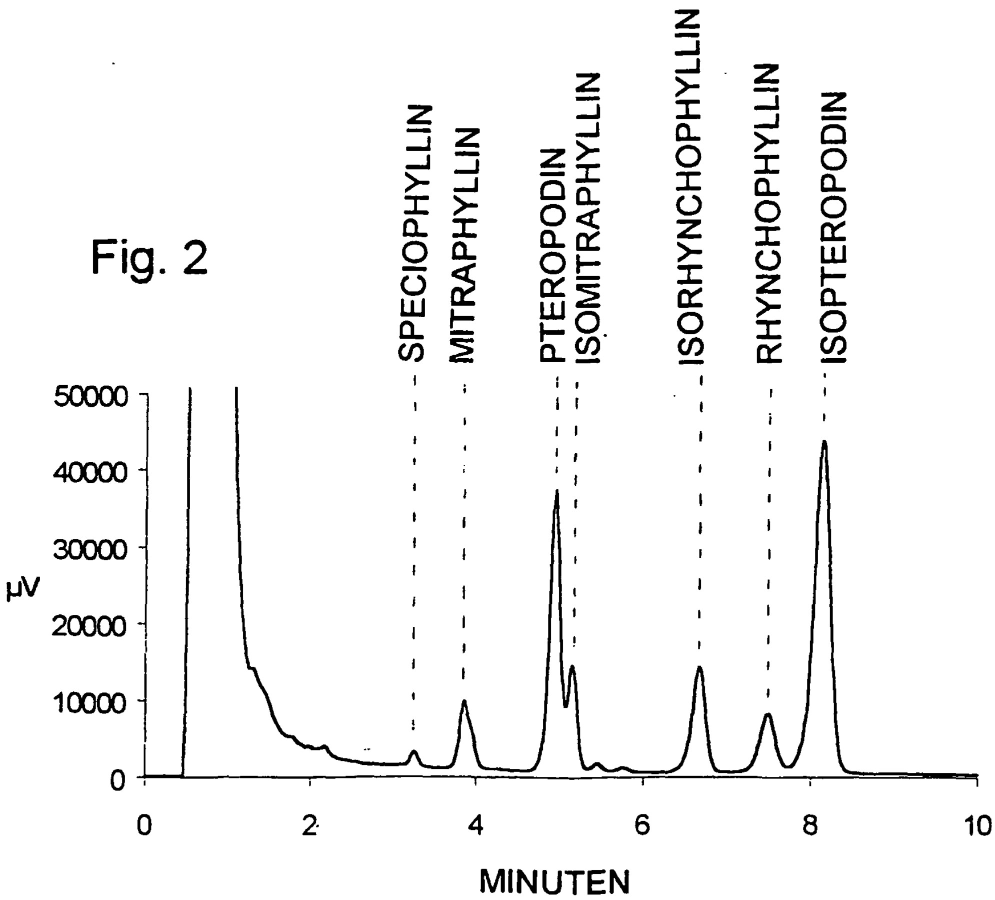
SPECIOPHYLLIN
MITRAPHYLLIN
PTEROPODIN
ISOMITRAPHYLLIN
ISORHYNCHOPHYLLIN
RHYNCHOPHYLLIN
ISOPTEROPODIN
50000
40000
30000
μV
20000
10000
0
0
2
4
6
8
10
MINUTEN

Fig. 3

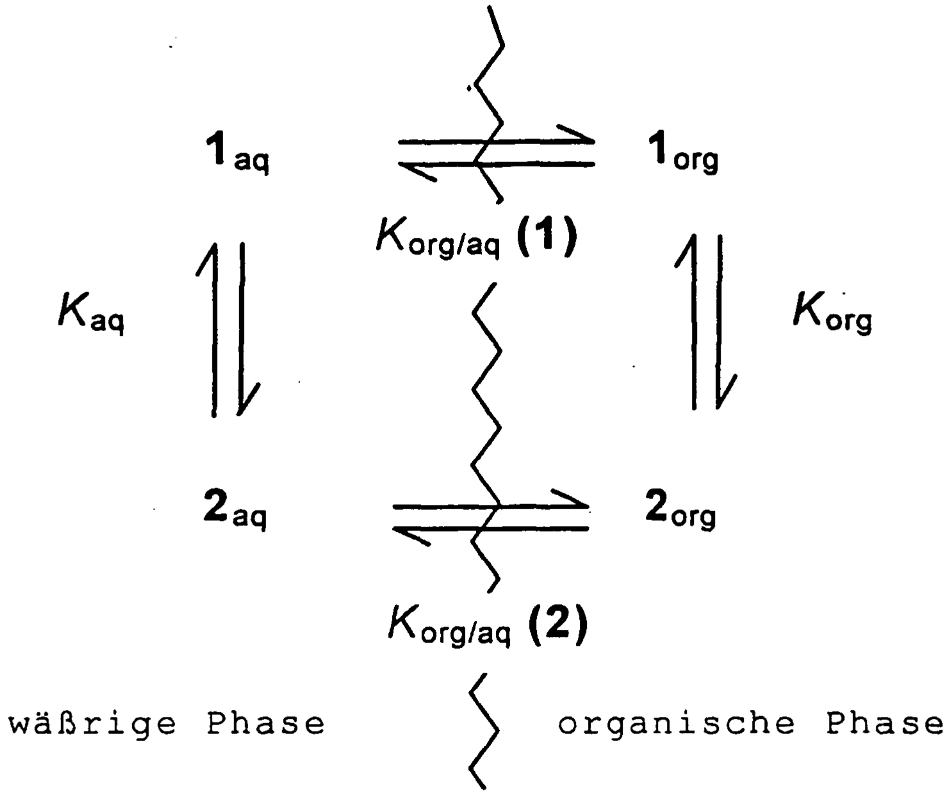
1aq
1org
Korg/aq (1)
Kaq
Korg
2aq
2org
Korg/aq (2)
wäßrige Phase
organische Phase

Fig. 4

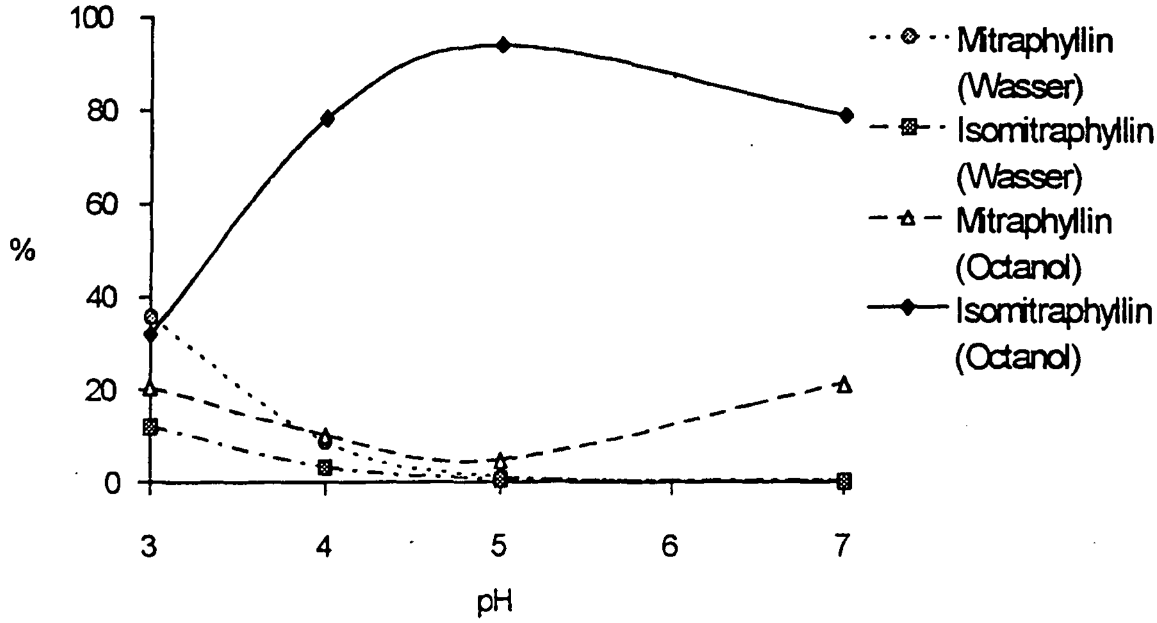
100
80
60
%
40
20
0
3
4
5
6
7
pH
Mitraphyllin (Wasser)
Isomitraphyllin (Wasser)
Mitraphyllin (Octanol)
Isomitraphyllin (Octanol)

Fig. 5

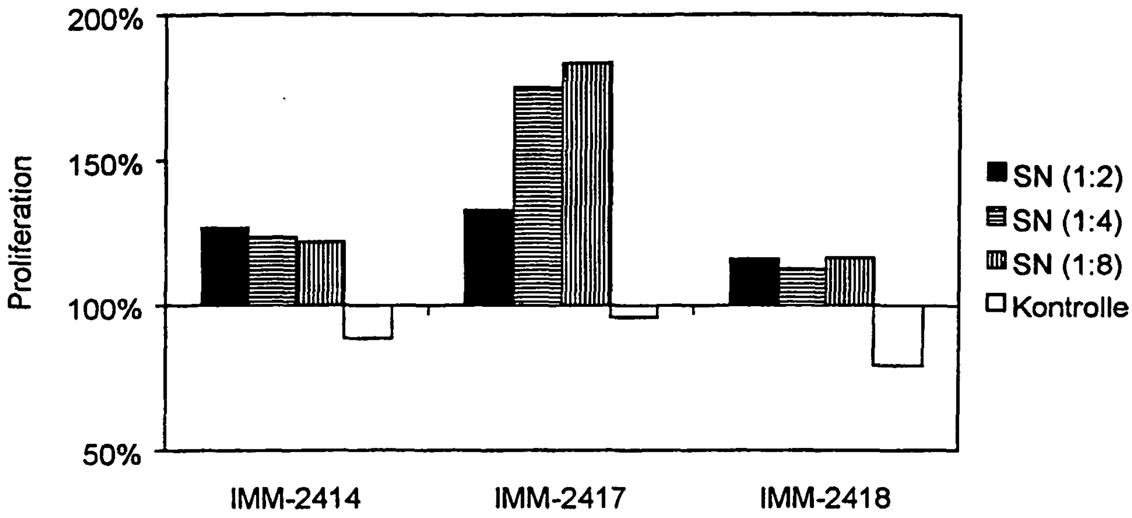
200%
150%
100%
50%
Proliferation
IMM-2414
IMM-2417
IMM-2418
SN (1:2)
SN (1:4)
SN (1:8)
Kontrolle

Fig. 6

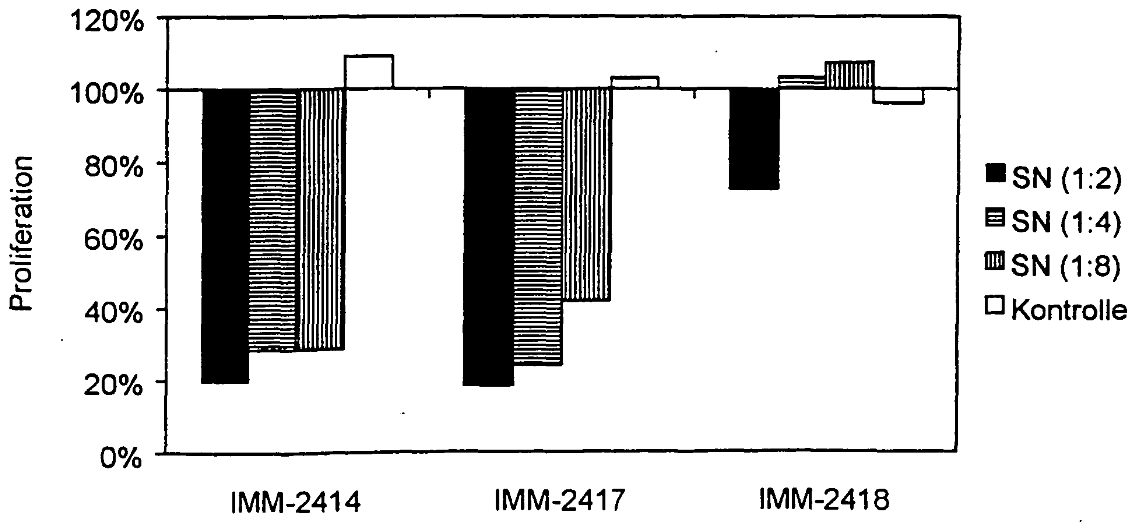
120%
100%
80%
60%
40%
20%
0%
Proliferation
IMM-2414
IMM-2417
IMM-2418
SN (1:2)
SN (1:4)
SN (1:8)
Kontrolle

Fig. 7

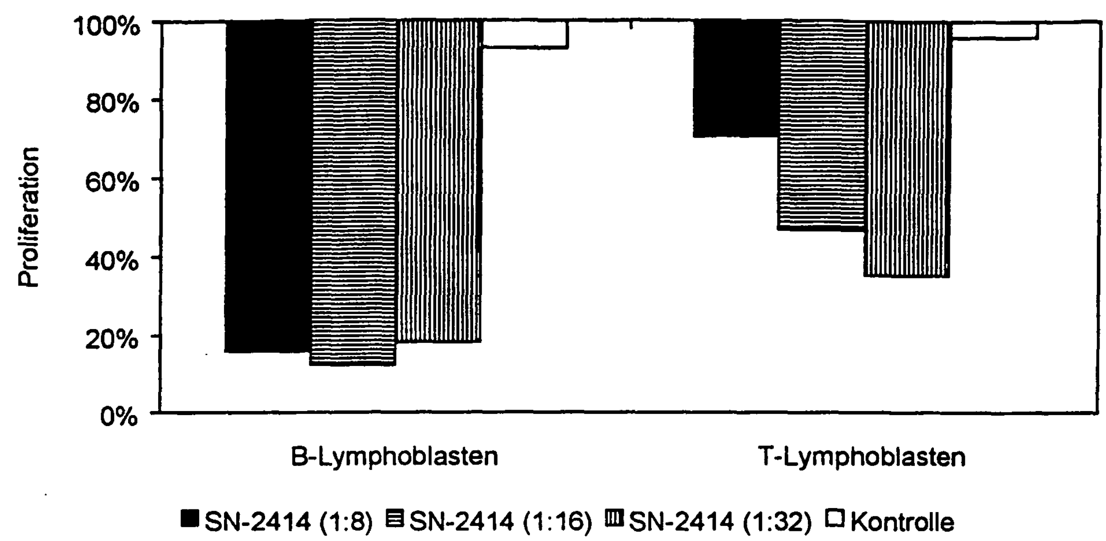
100%
80%
60%
40%
20%
0%
Proliferation
B-Lymphoblasten
T-Lymphoblasten
SN-2414 (1:8) SN-2414 (1:16) SN-2414 (1:32) Kontrolle

Fig. 8

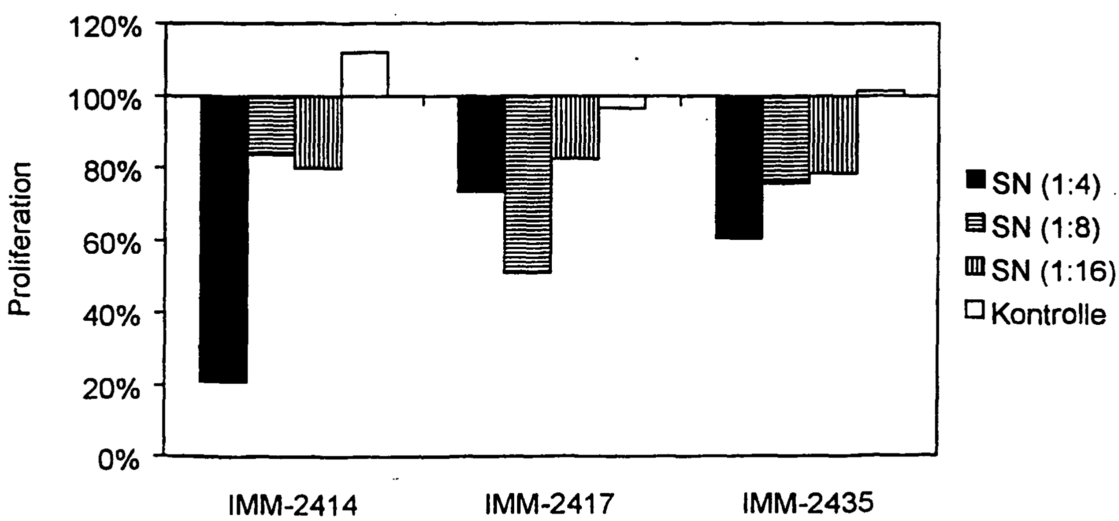
120%
100%
80%
60%
40%
20%
0%
Proliferation
IMM-2414
IMM-2417
IMM-2435
SN (1:4)
SN (1:8)
SN (1:16)
Kontrolle

Fig. 9

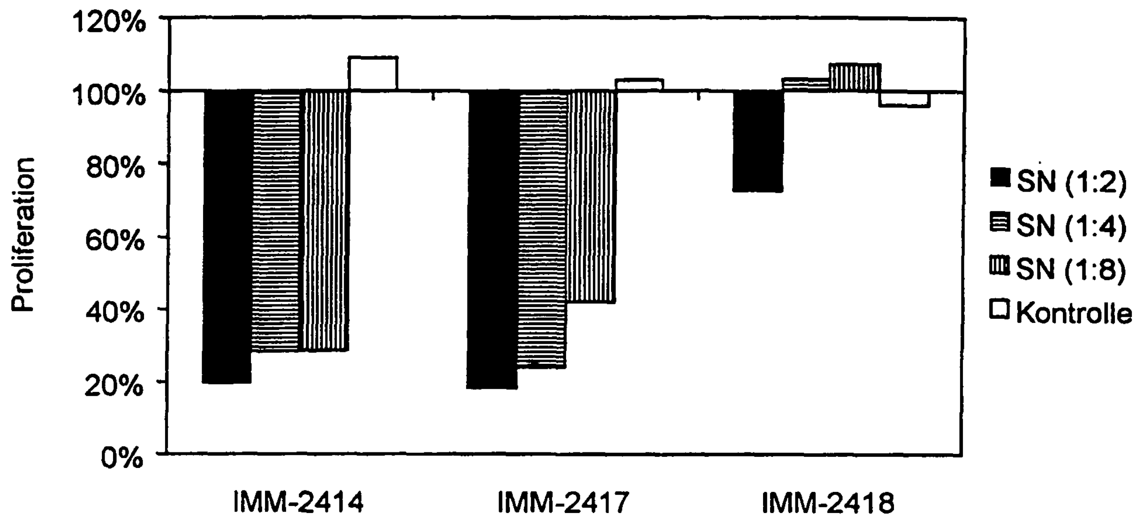
120%
100%
80%
60%
40%
20%
0%
Proliferation
IMM-2414
IMM-2417
IMM-2418
SN (1:2)
SN (1:4)
SN (1:8)
Kontrolle

Fig. 10

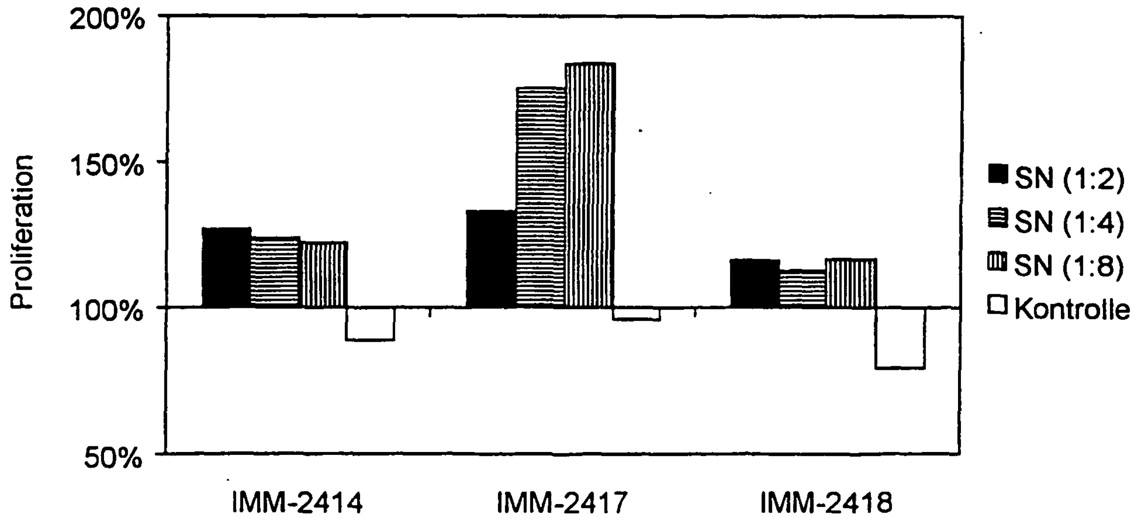
200%
150%
100%
50%
Proliferation
IMM-2414
IMM-2417
IMM-2418
SN (1:2)
SN (1:4)
SN (1:8)
Kontrolle

Fig. 11

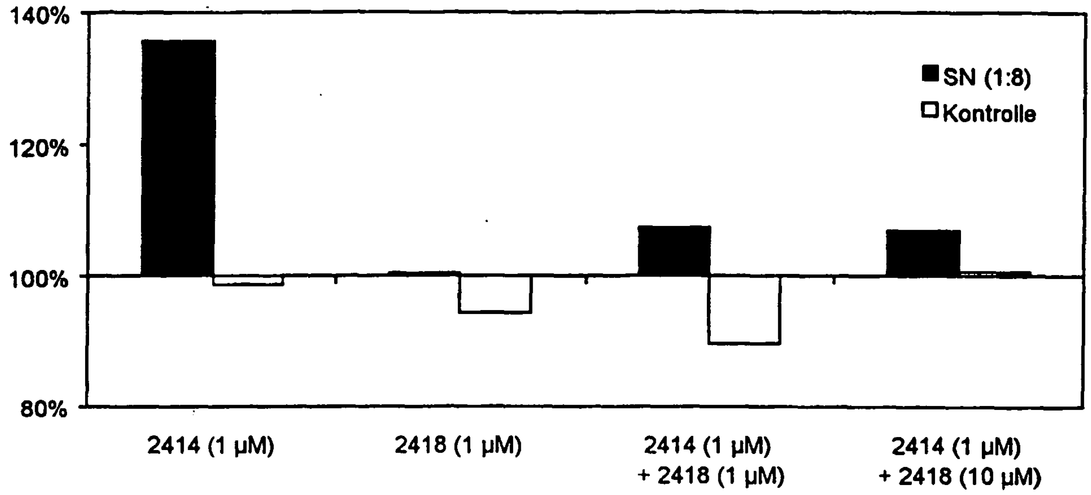
140%
120%
100%
80%
SN (1:8)
Kontrolle
2414 (1 µM)
2418 (1 µM)
2414 (1 µM) + 2418 (1 µM)
2414 (1 µM) + 2418 (10 µM)

Fig. 12

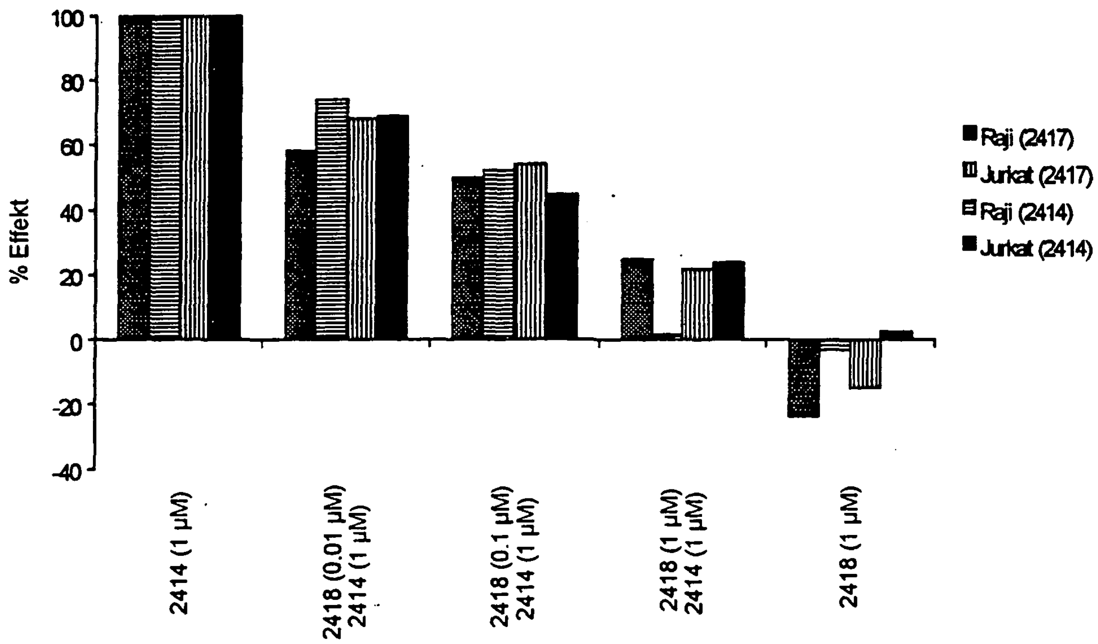
% Effekt
100
80
60
40
20
0
-20
-40
Raji (2417)
Jurkat (2417)
Raji (2414)
Jurkat (2414)
2414 (1 µM)
2418 (0.01 µM) 2414 (1 µM)
2418 (0.1 µM) 2414 (1 µM)
2418 (1 µM) 2414 (1 µM)
2418 (1 µM)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5302611 A
- WO 8600524 A


### In der Beschreibung aufgeführte Nicht-Patentliteratur


- **Wagner H. ; Kreutzkamp B. ; Jurcic K.** *Planta Med.,* 1985, vol. 51, 419-423
- **Stuppner H. ; Sturm S. ; Geisen G. ; Zillian U. ; Konwalinka G.** *Planta Med.,* 1993, vol. 59 (A 583
- **Kanatani H. ; Kohda H. ; Yamasaki K. ; Hotta I. ; Nakata Y. ; Segawa T. ; Yamanaka E. ; Aimi N. ; Sakai S.I.** *J. Pharm. Pharmacol.,* 1984, vol. 37, 401-404
- **Zhang W. ; Liu G.X.** *Act. Pharmacol. Sinica,* 1986, vol. 7 (5), 426-428
- **Zhu Y. ; Guoxiong H.X.** *Chin. J. Pharmacol. Toxicol.,* 1993, vol. 7 (2), 117-121
- **Sun A. ; Liu G. ; Wang X. ; Zhang W. ; Huang X.** *Chin. J. Pharmacol. Toxicol.,* 1988, vol. 2 (2), 93-97
- **Zhang W. ; Liu G. ; Huang X.** *Act. Pharmacol. Sinica,* 1987, vol. 8, 425-429
- **Jin R.M. ; Chen C.X. ; Li Y.K. ; Xu P.K.** *Act. Pharmaceut. Sinica,* 1991, vol. 26 (4), 246-249
- **Chen C.X. ; Jin R.M. ; Li Y.K. ; Zhong J. ; Yue L. ; Chen S.C. ; Zhou J.Y.** *Act. Pharmacol. Sinica,* 1992, vol. 13 (2), 126-130
- **Laus G. ; Brössner D. ; Senn G. ; Wurst K.** *J.Chem.Soc., Perkin Trans.,* 1996, vol. 2, 1931-1936
- **Laus G. ; Brössner D. ; Keplinger K.** *Phytochemistry,* 1997, vol. 45, 855-860
- **Laus G. ; Keplinger D.** *J. Chromatogr. A,* 1994, vol. 662, 243-249
- **Laus G. ; Keplinger K.** *Zeitschrift f. Phytotherapie,* 1997, 122-126
- **Edgell C.-J.S. ; McDonald C.C. ; Graham J.B.** *Proc. Natl. Acad. Sci. USA,* 1983, vol. 80, 3734-3737